# EUROPEAN PATENT SPECIFICATION

(11) **EP 2 590 631 B1**
(45) Date of publication and mention of the grant of the patent: **26.10.2016**
(21) Application number: 11736495.0
(22) Date of filing: 06.07.2011
(51) Int. Cl.: A61K 31/7042, A61K 9/24, A61K 31/381, A61K 9/20, A61K 31/155

(54) **FORMULATION FOR CO-THERAPY TREATMENT OF DIABETES**
FORMULIERUNG ZUR KOMBINIERTEN BEHANDLUNG VON DIABETES
FORMULATION POUR LE TRAITEMENT PAR CO-THERAPIE DU DIABETE

(30) Priority: 06.07.2010 US 361543 P
(43) Date of publication of application: 15.05.2013
(73) Proprietor: Janssen Pharmaceutica NV, 2340 Beerse (BE)
(72) Inventor: DELAET, Urbain, Alfons Clementina, 2340 Beerse (BE); FAURE, Anne, 2340 Beerse (BE); HEYNS, Philip, Erna Hortentia Gilbert, 2340 Beerse (BE); JANS, Eugeen, Maria Jozef, 2340 Beerse (BE); RAILKAR, Aniruddha, Ambler PA 19002 (US)
(74) Representative: Warner, James Alexander
(86) International application number: PCT/US2011/042988
(87) International publication number: WO 2012/006298

(56) References cited:
- WO-A1-00/28989
- WO-A2-2009/121945
- WO-A2-2010/045656

## Description

### FIELD OF THE INVENTION

The present invention is directed to a pharmaceutical compositions for co-therapy treatment and prevention of glucose-related disorders such as Type 2 diabetes mellitus and Syndrome X.

### BACKGROUND OF THE INVENTION

Diabetes mellitus is a medical term for the presence of elevated blood glucose. People with diabetes either don't produce insulin, produce too little insulin or do not respond to insulin, resulting in the build up of glucose in the blood. The most common form of diabetes is Type 2 diabetes, once referred to as adult onset diabetes or non-insulin dependent diabetes (NIDDM), which may account for >90% of diabetes in adults. However, as the younger population becomes increasingly overweight or obese, Type 2 diabetes is becoming more prevalent in teens and children. Diabetes may also refer to gestational diabetes, Type 1 diabetes or autoimmune diabetes, once referred to as juvenile onset diabetes and type 1 1/2 diabetes, also referred to as latent-autoimmune diabetes in adults or LADA. Diabetes may occur because of poor dietary habits or lack of physical activity (e.g., sedentary lifestyle), genetic mutations, injury to the pancreas, drug (e.g., AIDS therapies) or chemical (e.g., steroid) exposure or disease (e.g., cystic fibrosis, Down syndrome, Cushing's syndrome). Two rare types of genetic defects leading to diabetes are termed maturity-onset diabetes of the young (MODY) and atypical diabetes mellitus (ADM).

Type 2 diabetes mellitus (non-insulin-dependent diabetes mellitus or NIDDM) is a metabolic disorder involving disregulation of glucose metabolism and insulin resistance, and long-term complications involving the eyes, kidneys, nerves, and blood vessels. Type 2 diabetes mellitus usually develops in adulthood (middle life or later) and is described as the body's inability to make either sufficient insulin (abnormal insulin secretion) or its inability to effectively use insulin (resistance to insulin action in target organs and tissues). More particularly, patients suffering from Type 2 diabetes mellitus have a relative insulin deficiency. That is, in these patients, plasma insulin levels are normal to high in absolute terms, although they are lower than predicted for the level of plasma glucose that is present.

Type 2 diabetes mellitus is characterized by the following clinical signs or symptoms: persistently elevated plasma glucose concentration or hyperglycemia; polyuria; polydipsia and / or polyphagia; chronic microvascular complications such as retinopathy, nephropathy and neuropathy; and macrovascular complications such as hyperlipidemia and hypertension which can lead to blindness, end-stage renal disease, limb amputation and myocardial infarction.

Syndrome X, also termed Insulin Resistance Syndrome (IRS), Metabolic Syndrome, or Metabolic Syndrome X, is a disorder that presents risk factors for the development of Type 2 diabetes mellitus and cardiovascular disease including glucose intolerance, hyperinsulinemia and insulin resistance, hypertriglyceridemia, hypertension and obesity.

The diagnosis of Type 2 diabetes mellitus includes assessment of symptoms and measurement of glucose in the urine and blood. Blood glucose level determination is necessary for an accurate diagnosis. More specifically, fasting blood glucose level determination is a standard approach used. However, the oral glucose tolerance test (OGTT) is considered to be more sensitive than fasted blood glucose level. Type 2 diabetes mellitus is associated with impaired oral glucose tolerance (OGT). The OGTT thus can aid in the diagnosis of Type 2 diabetes mellitus, although generally not necessary for the diagnosis of diabetes (Emancipator K, Am J Clin Pathol 1999 Nov; 112(5):665-74; Type 2 Diabetes Mellitus, Decision Resources Inc., March 2000). The OGTT allows for an estimation of pancreatic beta-cell secretory function and insulin sensitivity, which helps in the diagnosis of Type 2 diabetes mellitus and evaluation of the severity or progression of the disease (e.g., Caumo A, Bergman RN, Cobelli C., J Clin Endocrinol Metab 2000, 85(11): 4396-402). More particularly, the OGTT is extremely helpful in establishing the degree of hyperglycemia in patients with multiple borderline fasting blood glucose levels that have not been diagnosed as diabetics. In addition, the OGTT is useful in testing patients with symptoms of Type 2 diabetes mellitus where the possible diagnosis of abnormal carbohydrate metabolism has to be clearly established or refuted.

Thus, impaired glucose tolerance is diagnosed in individuals that have fasting blood glucose levels less than those required for a diagnosis of Type 2 diabetes mellitus, but have a plasma glucose response during the OGTT between normal and diabetics. Impaired glucose tolerance is considered a pre-diabetic condition, and impaired glucose tolerance (as defined by the OGTT) is a strong predictor for the development of Type 2 diabetes mellitus (Haffner SM, Diabet Med 1997 Aug; 14 Suppl 3:S12-8).

Type 2 diabetes mellitus is a progressive disease associated with the reduction of pancreatic function and/or other insulin-related processes, aggravated by increased plasma glucose levels. Thus, Type 2 diabetes mellitus usually has a prolonged pre-diabetic phase and various pathophysiological mechanisms can lead to pathological hyperglycemia and impaired glucose tolerance, for instance, abnormalities in glucose utilization and effectiveness, insulin action and/or insulin production in the prediabetic state (Goldberg RB, Med Clin North Am 1998 Jul; 82(4):805-21).

The pre-diabetic state associated with glucose intolerance can also be associated with a predisposition to abdominal obesity, insulin resistance, hyperlipidemia, and high blood pressure, that is, Syndrome X (Groop L, Forsblom C, Lehtovirta M, Am J Hypertens 1997 Sep;10(9 Pt 2):172S-180S; Haffner SM, J Diabetes Complications 1997 Mar-Apr;11(2):69-76; Beck-Nielsen H, Henriksen JE, Alford F, Hother-Nielson O, Diabet Med 1996 Sep;13(9 Suppl 6):S78-84).

Thus, defective carbohydrate metabolism is pivotal to the pathogenesis of Type 2 diabetes mellitus and impaired glucose tolerance (Dinneen SF, Diabet Med 1997 Aug; 14 Suppl 3:S19-24). In fact, a continuum from impaired glucose tolerance and impaired fasting glucose to definitive Type 2 diabetes mellitus exists (Ramlo-Halsted BA, Edelman SV, Prim Care 1999 Dec; 26(4):771-89).

Early intervention in individuals at risk to develop Type 2 diabetes mellitus, focusing on reducing the pathological hyperglycemia or impaired glucose tolerance may prevent or delay the progression towards Type 2 diabetes mellitus and associated complications and/or Syndrome X. Therefore, by effectively treating impaired oral glucose tolerance and / or elevated blood glucose levels, one can prevent or inhibit the progression of the disorder to Type 2 diabetes mellitus or Syndrome X.

Typical treatment of glucose disorders including Type 2 diabetes mellitus and Syndrome X focuses on maintaining the blood glucose level as near to normal as possible and includes diet and exercise, and when necessary, treatment with anti-diabetic agents, insulin or a combination thereof. Type 2 diabetes mellitus that cannot be controlled by dietary management is treated with oral antidiabetic agents including, but not limited to, sulfonylureas (e.g., not limited to first generation: chlorpropamide, tolazamide, tolbutamide; second generation: glyburide, glipizide; and third generation: glimepiride), biguanides (e.g., metformin), thiazolidinediones (e.g., rosiglitazone, pioglitazone, troglitazone), alpha-glucosidase inhibitors (e.g., acarbose, miglitol), meglitinides (e.g., repaglinide), other insulin-sensitizing compounds, and /or other anti-obesity agents (e.g., orlistat or sibutramine). For Syndrome X, the anti-diabetic agents are additionally combined with pharmacological agents for the treatment of the concomitant co-morbidities (e.g., antihypertensives for hypertension, hypolipidemic agents for hyperlipidemia).

First-line therapies typically include metformin and sulfonylureas as well as thiazolidinediones. Metformin monotherapy is a first line choice, particularly for treating Type 2 diabetic patients who are also obese and / or dyslipidemic. Lack of an appropriate response to metformin is often followed by treatment with metformin in combination with sulfonylureas, thiazolidinediones, or insulin. Sulfonylurea monotherapy (including all generations of drugs) is also a common first line option. Another first line therapy choice may be thiazolidinediones. Patients who do not respond appropriately to oral anti-diabetic monotherapy, are given combinations of these agents. When glycemic control cannot be maintained with oral antidiabetics alone, insulin therapy is used either as a monotherapy, or in combination with oral antidiabetic agents. These same strategies, optionally in combination with additional strategies (e.g., anti-hypertensive) can be used for the treatment of Syndrome X.

Anti-diabetic agents include, but are not limited to:
(a) Sulfonylureas, which increase insulin production by stimulating pancreatic beta cells, and therefore act as insulin secretagogues. The primary mechanism of action of sulfonylureas is to close ATP-sensitive potassium channels in the beta-cell plasma membrane, initiating a chain of events that result in insulin release. Suitable examples of sulfonylureas include, but are not limited to chlorpropamide, tolazamide, tolbutamide, glyburide, glipizide, glimepiride, and like.
(b) Meglitinides, another class of insulin secretagogues, that have a mechanism of action distinct from that of the sulfonylureas. Suitable examples of meglitinides include, but are not limited to repaglinide.
(c) Agents which modify insulin secretion such as Glucagon-like Peptide-1 (GLP-1) and it's mimetics, Glucose-insulinotropic peptide (GIP) and it's mimetics, Exendin and it's mimetics, and Dipeptyl Protease Inhibitors (DPPIV).
(d) Biguanides which decrease liver glucose production and increase the uptake of glucose. Suitable examples include, but are not limited to metformin.
(e) Thiazolidinediones, insulin sensitizing drugs which decrease peripheral insulin resistance by enhancing the effects of insulin at target organs and tissues. These drugs bind and activate the nuclear receptor, peroxisome proliferator-activated receptor-gamma (PPAR-gamma) which increases transcription of specific insulin-responsive genes. Suitable examples of PPAR-gamma agonists are the thiazolidinediones which include, but are not limited to rosiglitazone, pioglitazone, troglitazone, isaglitazone (known as MCC-555), 2-[2-[(2R)-4-hexyl-3,4-dihydro-3-oxo-2*H*-1,4-benzoxazin-2-yl]ethoxy]benzene acetic acid, and the like. Additionally, the non-thiazolidinediones also act as insulin sensitizing drugs, and include, but are not limited to GW2570, and the like.
(f) Retinoid-X receptor (RXR) modulators, also insulin sensitizing drugs, which include, but are not limited to targretin, 9-cis-retinoic acid, and the like.
(g) Other insulin sensitizing agents include, but are not limited to INS-1, PTP-1 B inhibitors, GSK3 inhibitors, glycogen phosphorylase inhibitors, fructose-1,6-bisphosphatase inhibitors, and the like.
(h) Alpha-glucosidase inhibitors which act to inhibit alpha-glucosidase. Alpha-glucosidase converts fructose to glucose, thus these inhibitors delay the digestion of carbohydrates. The undigested carbohydrates are subsequently broken down in the gut, thereby reducing the post-prandial glucose peak. Suitable examples include, but are not limited to, acarbose and miglitol.
(i) Insulins, including regular or short-acting, intermediate-acting, and long-acting insulins, inhaled insulin and insulin analogues such as insulin molecules with minor differences in the natural amino acid sequence. These modified insulins may have faster onset of action and / or shorter duration of action.
(j) Small molecule mimics of insulin, including, but not limited to L-783281, TE-17411, and the like.
(k) Na-glucose co-transporter inhibitors which inhibit the renal reabsorption of glucose such as T-1095, T-1095A, phlorizen, and the like.
(l) Amylin agonists which include, but are not limited to pramlintide, and the like.
(m) Glucagon antagonists such as AY-279955, and the like.

In addition to antidiabetic agents, therapies may include add-on treatment with anti-obesity agents such as orlistat, a pancreatic lipase inhibitor, which prevents the breakdown and absorption of fat; or sibutramine, an appetite suppressant and inhibitor of the reuptake of serotonin, norepinephrine and dopamine in the brain. Other potential add-on anti-obesity agents include, but are not limited to, appetite-suppressants acting through adrenergic mechanisms such as benzphetamine, phenmetrazine, phentermine, diethylpropion, mazindol, sibutramine, phenylpropanolamine or, ephedrine; appetite-suppressant agents acting through serotonergic mechanisms such as quipazine, fluoxetine, sertraline, fenfluramine, or dexfenfluramine; appetite-suppressant agents acting through dopamine mechanisms, eg, apomorphine; appetite-suppressant agents acting through histaminergic mechanisms (eg, histamine mimetics, H3 receptor modulators); enhancers of energy expenditure such as beta-3 adrenergic agonists and stimulators of uncoupling protein function; leptin and leptin mimetics; neuropeptide Y antagonists; melanocortin-1, 3 and 4 receptor modulators; cholecystokinin agonists; glucagon-like peptide-1 (GLP-1) mimetics and analogues (eg, Exendin); androgens (eg, dehydroepiandrosterone and derivatives such as etiocholandione), testosterone, anabolic steroids (eg, oxandrolone), and steroidal hormones; galanin receptor antagonists; cytokine agents such as ciliary neurotrophic factor; amylase inhibitors; enterostatin agonists/mimetics; orexin/hypocretin antagonists; urocortin antagonists; bombesin agonists; modulators of protein kinase A; corticotropin-releasing factor mimetics; cocaine- and amphetamine-regulated transcript mimetics; calcitonin-gene related peptide mimetics; and fatty acid synthase inhibitors.

There remains a need to provide an effective treatment for glucose related disorders such as elevated glucose levels, Type 2 diabetes mellitus, Syndrome X, and the like. There also remains a need to provide an effective treatment for glucose related disorders which also slows or prevents the progression and / or development of Type 2 diabetes mellitus.

### SUMMARY OF THE INVENTION

The present invention is directed to a pharmaceutical composition wherein the pharmaceutical composition is a tablet comprising:
(a) an extended release layer comprising metformin hydrochloride and a controlled release excipient comprising a mixture of two carbomers; and
(b) an immediate release layer comprising a crystalline hemihydrate form of a compound of formula (I-X) 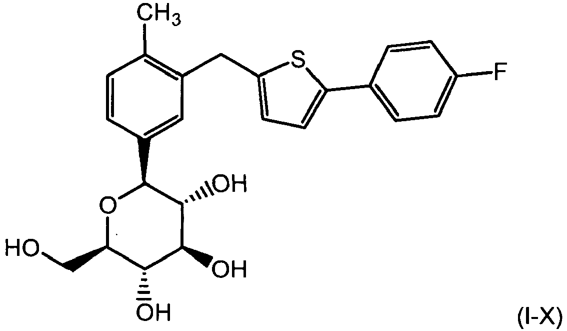
or pharmaceutically acceptable salt thereof.

The present invention is further directed to methods for the preparation of the pharmaceutical compositions of the present invention, as described in more detail hereinafter.

The present invention is further directed to the composition of the invention for use in methods of co-therapy for the treatment and / or prevention of glucose-related disorders, said methods comprising administering to a subject in need thereof any of the pharmaceutical compositions as described herein.

### BRIEF DESCRIPTION OF THE FIGURES

Figure 1 illustrates measured dissolution profiles for metformin HCl, from mono-layer and bi-layer tablet compositions prepared as described in Example 1, compared with 2 tablets 500 mg GLUCOPHAGE^{®} XR.
Figure 2 illustrates measured dissolution profiles for metformin HCl, from bi-layer tablets prepared as described in Example 2, compared with 1 and 2 tablets of 500 mg GLUCOPHAGE^{®} XR.
Figure 3 illustrates measured dissolution profiled for the compound of formula (I-X), from bi-layer tablets prepared as described in Example 2.
Figure 4 illustrates measured dissolution profiles for metformin HCl, from bi-layer tablets prepared as described in Example 3, compared with 1 tablet of 500 mg GLUCOPHAGE^{®} XR.

### DETAILED DESCRIPTION OF THE INVENTION

The present invention is directed to a pharmaceutical composition, wherein the pharmaceutical composition is a tablet comprising
(a) an extended release layer comprising metformin or a pharmaceutically acceptable salt thereof, preferably metformin hydrochloride; and a controlled release excipient comprising a mixture of two carbomers; and
(b) an immediate release layer comprising a crystalline hemihydrate form of compound of formula (I-X) 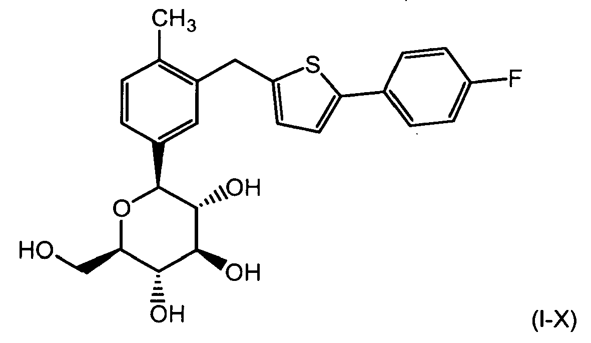 or pharmaceutically acceptable salt thereof.

The compound of the formula (I-X) exhibits an inhibitory activity against sodium-dependent glucose transporter, such as for example SGLT2. The compounds of formula (I-X) may be prepared according to the process as disclosed in Nomura, S. et al., US Patent Publication, US 2005/0233988 A1, published October 20, 2005, which is incorporated by reference herein. The compound of formula (I-X) may also be referred to as 1-(β-D-glucopyranosyl)-4-methyl-3-[5-(4-fluorophenyl)-2-thienylmethyl]benzene.

In certain preferred embodiments, the compound of formula (I-X) is the crystalline form of the hemihydrate of the compound of formula (I-X), as described in WO 2008/069327, the disclosure of which is hereby incorporated by reference in its entirety. The hemihydrate of the compound of Formula (I-X) may also be referred to as 1-(β-D-glucopyranosyl)-4-methyl-3-[5-(4-fluorophenyl)-2-thienylmethyl]benzene hemihydrate.

The pharmaceutically acceptable salt of the compounds of the formula (I-X) include, for example, a salt with an alkali metal such as lithium, sodium, potassium, etc.; a salt with an alkaline earth metal such as calcium, magnesium, etc.; a salt with zinc or aluminum; a salt with an organic base such as ammonium, choline, diethanolamine, lysine, ethylenediamine, t-butylamine, t-octylamine, tris(hydroxymethyl)aminomethane, N-methyl glucosamine, triethanolamine and dehydroabietylamine; a salt with an inorganic acid such as hydrochloric acid, hydrobromic acid, hydroiodic acid, sulfuric acid, nitric acid, phosphoric acid, etc.; or a salt with an organic acid such as formic acid, acetic acid, propionic acid, oxalic acid, malonic acid, succinic acid, fumaric acid, maleic acid, lactic acid, malic acid, tartaric acid, citric acid, methanesulfonic acid, ethanesulfonic acid, benzenesulfonic acid, etc.; or a salt with an acidic amino acid such as aspartic acid, glutamic acid, etc.

The compound of formula (I-X) also includes a mixture of stereoisomers, or each pure or substantially pure isomer. In addition, the compounds of formula (I-X) include an intramolecular salt, hydrate, solvate or polymorphism thereof.

Metformin, and more particularly metformin hydrochloride, (also known by the trade names GLUCOPHAGE^{®}, RIOMET^{®}, FORTAMET^{®}, GLUMETZA^{®}, OBIMET^{®}, and others) is an oral anti-diabetic drug of the biguanide class. Metformin is a first-line therapy for Type 2 diabetes mellitus, particularly in overweight and obese people. The usual starting dose of metformin (for example, as metformin hydrochloride tablets) in the United States and certain other countries is 500 mg twice a day or 850 mg once a day, given with meals. The daily dosage may be increased in increments of 500 mg weekly or 850 mg every 2 weeks, up to a total of 2000 mg per day, given in divided doses. Patients can also be titrated from 500 mg twice a day to 850 mg twice a day after 2 weeks. For those patients requiring additional glycemic control, metformin may be given to a maximum recommended daily dose of e.g., 2550 mg per day. Doses above 2000 mg may be better tolerated given three times a day with meals. Preferably, the metformin or pharmaceutically acceptable salt thereof is metformin hydrochloride.

In an embodiment, the present invention is directed to a pharmaceutical composition wherein the metformin or pharmaceutically acceptable salt thereof is metformin hydrochloride. In another embodiment, the present invention is directed to a pharmaceutical composition wherein the metformin hydrochloride is present at a dosage amount in the range of from about 100 mg to about 2000 mg, preferably from about 250 mg to about 2000 mg, preferably from about 500 mg to about 1000 mg, or any amount or range therein. In another embodiment, the present invention is directed to a pharmaceutical composition wherein the metformin hydrochloride is present at a dosage amount selected from the group consisting of 250 mg, 500 mg, 750 mg, 850 mg, 1000 mg, 1700 mg and 2000 mg.

In another embodiment, the present invention is directed to a pharmaceutical composition wherein the compound of formula (I-X) or pharmaceutically acceptable salt thereof is present at a dosage amount in the range of from about 1 mg to about 1000 mg, preferably from about 10 mg to about 500 mg, preferably from about 25 mg to about 500 mg, or any amount or range therein. In another embodiment, the present invention is directed to a pharmaceutical composition wherein the compound of formula (I-X) or pharmaceutically acceptable salt thereof is present at a dosage amount in the range of from about 25 mg to about 300 mg, preferably selected from the group consisting of 50 mg, 75 mg, 100 mg, 150 mg, 200 mg, 300 mg and 500 mg.

In another embodiment, the present invention is directed to a bi-layer tablet comprising:
(a) an extended release layer comprising metformin or a pharmaceutically acceptable salt thereof (preferably metformin hydrochloride); wherein the metformin or pharmaceutically acceptable salt thereof is present in an amount in the range of from about 100 mg to about 2000 mg, preferably from about 500 mg to about 1000 mg, or any amount or range therein; and
(b) an immediate release layer comprising a compound of formula (I-X) or pharmaceutically acceptable salt thereof; wherein the compound of formula (I-X) or pharmaceutically acceptable salt thereof is present in an amount in the range of from about 1 mg to about 1000 mg, or any amount or range therein (preferably, in an amount in the range of from about 10 mg to about 500 mg, or any amount or range therein, more preferably in an amount in the range of from about 50 mg to about 500 mg, or any amount or range therein.

In certain embodiments, the present invention is directed to a pharmaceutical composition, preferably a solid oral dosage form, more preferably a tablet, more preferably a bi-layer tablet, comprising (a) an extended release layer comprising metformin hydrocholoride; and (b) an immediate release layer comprising a compound of formula (I-X) or pharmaceutically acceptable salt thereof.

In an embodiment, the present invention is directed to a bi-layer tablet comprising (a) an extended release layer comprising metformin HCl and (b) an immediate release layer comprising a crystalline hemihydrate form of the compound of formula (I-X).

In an embodiment, the present invention is directed to a pharmaceutical composition comprising (a) an extended release layer comprising metformin hydrochloride; (b) an immediate release layer comprising a compound of formula (I-X) or pharmaceutically acceptable salt thereof; and wherein the extended release layer and the immediate release layer each further comprise one or more pharmaceutically acceptable excipients, as described in more detail herein.

Pharmaceutically acceptable excipients, include but are not limited to disintegrants, binders, diluents, lubricants, stabilizers, antioxidants, osmotic agents, colorants, plasticizers, coatings and the like. More particularly, suitable pharmaceutical excipients comprise one or more of the following: (i) diluents such as lactose, microcrystalline cellulose, dicalcium phosphate, starch and the like; (ii) binders such as polyvinylpyrrolidone (such as POVIDONE), methylcellulose, hydroxypropyl cellulose, hydroxypropyl methylcellulose (such as METHOCEL™ E-5), and the like; (iii) disintegrants such as sodium starch glycolate, croscamellose sodium, crospovidone and the like; (iv) wetting agents such as surfactants, such as sodium lauryl stearate, polysorbate 20, and the like; (v) lubricants such as magnesium stearate, sodium stearyl fumarate, talc, and the like; (vi) flow promoters or glidants such as colloidal silicon dioxide, talc and the like; and other excipients known to be useful in the preparation of pharmaceutical compositions. Additional suitable pharmaceutical excipients and their properties may be found in texts such as Handbook of Pharmaceutical Excipients, Edited by R.C. Rowe, P.J. Sheskey & P.J. Weller, Fourth Edition (Published by Pharmaceutical Press, a Division of Royal Pharmaceutical Society of Great Britain).

Fillers or diluents for use in the pharmaceutical compositions of the present invention include fillers or diluents typically used in the formulation of pharmaceuticals. Examples of fillers or diluents for use in accordance with the present invention include but are not limited to sugars such as lactose, dextrose, glucose, sucrose, cellulose, starches and carbohydrate derivatives, polysaccharides (including dextrates and maltodextrin), polyols (including mannitol, xylitol, and sorbitol), cyclodextrins, calcium carbonates, magnesium carbonates, microcrystalline cellulose, combinations thereof, and the like. In certain preferred embodiments the filler or diluent is lactose, microcrystalline cellulose, or combination thereof. Several types of microcrystalline cellulose are suitable for use in the formulations described herein, for example, microcrystalline cellulose selected from the group consisting of Avicel® types: PH101, PH102, PH103, PH105, PH 112, PH113, PH200, PH301, and other types of microcrystalline cellulose, such as silicified microcrystalline cellulose. Several types of lactose are suitable for use in the formulations described herein, for example, lactose selected from the group consisting of anhydrous lactose, lactose monohydrate, lactose fast flo, directly compressible anhydrous lactose, and modified lactose monohydrate.

Binders for use in the pharmaceutical compositions of the present invention include binders commonly used in the formulation of pharmaceuticals. Examples of binders for use in accordance with the present invention include but are not limited to cellulose derivatives (including hydroxypropyl cellulose, hydroxypropyl methylcellulose, methylcellulose, and sodium carboxymethyl cellulose), glycol, sucrose, dextrose, corn syrup, polysaccharides (including acacia, targacanth, guar, alginates and starch), corn starch, pregelatinized starch, modified corn starch, gelatin, polyvinylpyrrolidone, polyethylene, polyethylene glycol, combinations thereof and the like.

Disintegrants for use in the pharmaceutical compositions of the present invention include disintegrants commonly used in the formulation of pharmaceuticals. Examples of disintegrants for use in accordance with the present invention include but are not limited to starches, and crosslinked starches, celluloses and polymers, combinations thereof and the like. Representative disintegrants include microcrystalline cellulose, croscarmellose sodium, alginic acid, sodium alginate, crosprovidone, cellulose, agar and related gums, sodium starch glycolate, corn starch, potato starch, sodiumstarch glycolate, Veegum HV, methylcellulose, agar, bentonite, sodium carboxymethylcellulose, calcium carboxymethylcellulose, carboxymethylcellulose, alginic acid, guar gum combinations thereof, and the like.

Lubricants, glidants or anti-tacking agents for use in the pharmaceutical compositions of the present invention include lubricants, glidants and anti-tacking agents commonly used in the formulation of pharmaceuticals. Examples for use in accordance with the present invention include but are not limited to magnesium carbonate, magnesium laurylsulphate, calcium silicate, talc, fumed silicon dioxide, combinations thereof, and the like. Other useful lubricants include but are not limited to magnesium stearate, calcium stearate, stearic acid, sodium stearyl fumarate, polyethylene glycol, sodium lauryl sulphate, magnesium lauryl sulphate, sodium benzoate, colloidal silicon dioxide, magnesium oxide, magnesium silicate, mineral oil, hydrogenated vegetable oils, waxes, glyceryl behenate, polyethylene glycol, and combinations thereof, and the like.

Surfactants for use in the pharmaceutical compositions of the present invention include surfactants commonly used in the formulation of pharmaceuticals. Examples of surfactants for use in accordance with the present invention include but are not limited to ionic-and nonionic surfactants or wetting agents commonly used in the formulation of pharmaceuticals, such as ethoxylated castor oil, polyglycolyzed glycerides, acetylated monoglycerides, sorbitan fatty acid esters, poloxamers, polyoxyethylene sorbitan fatty acid esters, polyoxyethylene derivatives, monoglycerides or ethoxylated derivatives thereof, diglycerides or polyoxyethylene derivatives thereof, sodium docusate, sodium laurylsulfate, cholic acid or derivatives thereof, lecithins, phospholipids, combinations thereof, and the like.

Other polymers commonly which may be used as excipients in the pharmaceutical compositions of the present invention include, but are not limited to, methylcellulose (MC), ethylcellulose (EC), hydroxyethylcellulose (HEC), methyl hydroxyethylcellulose (MHEC), hydroxypropyl cellulose (HPC), hydroxypropyl methylcellulose (HPMC), sodium carboxymethylcellulose (NaCMC), and the like. These polymers, either alone or in various combinations, may serve multiple purposes including but not limited to controlling release of the compound of the formulations of the present invention.

The pharmaceutical compositions disclosed herein can further comprise antioxidants and chelating agents. For example, the pharmaceutical formulations can comprise butylated hydroxyanisole (BHA), butylated hydroxytoluene (BHT), propyl gallate (PG), sodium metabisulfite, ascorbyl palmitate, potassium metabisulfite, disodium EDTA (ethylenediamine tetraacetic acid; also known as disodium edentate), EDTA, tartaric acid, citric acid, citric acid monohydrate, and sodium sulfite.

The pharmaceutical compositions disclosed herein can further comprise one or more flow regulators (or glidants). Flow regulators may be present in powders or granules and are admixed in order to increase their flowability of the composition during manufacture, particularly in the preparation of tablets produced by pressing powders or granules. Flow regulators which can be employed include, but are not limited to, highly disperse silicon dioxide (Aerosil) or dried starch.

The tablet compositions of the present invention may further comprise a coating. Suitable coatings are film-forming polymers, such as, for example, those from the group of the cellulose derivatives, dextrins, starches, natural gums, such as, for example, gum arabic, xanthans, alginates, polyvinyl alcohol, polymethacrylates and derivatives thereof, such as, for example, Eudragit®, which may be applied to the tablet as solutions or suspensions by means of the various pharmaceutical conventional methods, such as, for example, film coating. The coating is typically applied as a solutions/suspensions which, in addition to any film-forming polymer present, may further comprise one or more adjuvants, such as hydrophilisers, plasticisers, surfactants, dyes and white pigments, such as, for example, titanium dioxide.

One skilled in the art will readily recognize that the appropriate pharmaceutically acceptable excipients are selected such that they are compatible with other excipients and do not bind with the drug compound(s) (active ingredient(s)) or cause drug degradation.

In certain embodiments of the present invention, the pharmaceutical composition preferably comprises between about 5% and about 50% by weight of diluents (relative to the total weight of the tablet or any individual extended release or immediate release layer), more preferably between about 5% and about 25% by weight diluent, more preferably still about 7% diluent.

In additional embodiments of the present invention, the pharmaceutical composition preferably comprises between about 1% and about 10% by weight of binder (relative to the total weight of the tablet or any individual extended release or immediate release layer), more preferably between about 3% and about 5% by weight binder, more preferably still about 4% binder.

In additional embodiments of the present invention, the pharmaceutical composition preferably comprises between about 1% and about 10% by weight of disintegrant (relative to the total weight of the tablet or any individual extended release or immediate release layer), more preferably between about 2% and about 5% by weight disintegrant, more preferably still about 3% disintegrant.

In additional embodiments of the present invention, the pharmaceutical composition preferably comprises between about 0% and about 5% by weight of wetting agent (relative to the total weight of the tablet or any individual extended release or immediate release layer), more preferably between about 0.1 % and about 2% by weight wetting agent, more preferably still about 0.3% wetting agent.

In additional embodiments of the present invention, the pharmaceutical composition preferably comprises between about 0% and about 3% by weight of lubricant (relative to the total weight of the tablet or any individual extended release or immediate release layer), more preferably between about 0.1 % and about 2% by weight lubricant, more preferably still about 0.5% lubricant.

### BILAYER / BILAYER FORMULATION

### IMMEDIATE RELEASE LAYER:

In an embodiment of the present invention, the immediate release layer comprises a compound of formula (I-X) or pharmaceutically acceptable salt thereof, preferably in an amount in the range of from about 50 mg to about 500 mg, or any amount or range thereof, more preferably in an amount in the range of from about 100 mg to about 300 mg, or any amount or range therein, more preferably, in an amount of about 50 mg or about 150 mg. In an embodiment of the present invention, the compound of formula (I-X), is present as its corresponding hemihydrate; and is further is present in an amount in the range of from about 50 mg to about 500 mg, or any amount or range therein, preferably in an amount in the range of from about 100 mg to about 300 mg, or any amount or range therein. Preferably in an amount of about 51 mg, about 102 mg, about 153 mg, about 204 mg, or about 306 mg, more preferably about 51 mg or about 153 mg. One skilled in the art will recognize that wherein the compound of formula (I-X) is present as its corresponding hemihydrate, the amount of the compound of 1-(β-D-glucopyranosyl)-4-methyl-3-[5-(4-fluorophenyl)-2-thienylmethyl]benzene hemihydrate is adjusted to provide the desired equivalent amount of 1-(β-D-glucopyranosyl)-4-methyl-3-[5-(4-fluorophenyl)-2-thienylmethyl]benzene. Thus for example, about 153 mg of 1-(β-D-glucopyranosyl)-4-methyl-3-[5-(4-fluorophenyl)-2-thienylmethyl]benzene hemihydrate is used to provide a composition comprising about 150 mg of 1-(β-D-glucopyranosyl)-4-methyl-3-[5-(4-fluorophenyl)-2-thienylmethyl]benzene. In another embodiment of the present invention, the immediate release layer further comprises one or more of the following additional components / excipients: (a) one or more fillers, (b) one or more binders, (c) one or more disintegrants and / or (d) one or more lubricants.

FILLER: In an embodiment of the present invention, the filler (in the immediate release layer) is microcrystalline cellulose, anhydrous lactose or a mixture thereof. In another embodiment of the present invention, the filler is present in an amount in the range of from about 25% by weight to about 55% by weight (relative to the total weight of the immediate release layer), or any amount or range therein, preferably in an amount in the range of from about 35% by weight to about 45% by weight, or any amount or range therein, more preferably in an amount of about 40% by weight.

BINDER: In an embodiment of the present invention, the binder (in the immediate release layer) is hydroxypropylcellulose. In another embodiment of the present invention, the binder is present in an amount in the range of from about 1 % by weight to about 5% by weight (relative to the total weight of the immediate release layer), or any amount or range therein, preferably in an amount in the range of from about 2% by weight to about 4% by weight, or any amount or range therein, more preferably in an amount of about 3% by weight.

DISINTEGRANT: In an embodiment of the present invention, the disintegrant (in the immediate release layer) is croscamellose sodium. In another embodiment of the present invention, the disintegrant is present in an amount in the range of from about 2% by weight to about 10% by weight (relative to the total weight of the immediate release layer), or any amount or range therein, preferably in an amount in the range of from about 4% by weight to about 7.5% by weight, or any amount or range therein, more preferably in an amount of about 6% by weight.

LUBRICANT: In an embodiment of the present invention, the lubricant (in the immediate release layer) is magnesium stearate. In another embodiment of the present invention, the lubricant is present in an amount in the range of from about 0.1 % by weight to about 2% by weight (relative to the total weight of the immediate release layer), or any amount or range therein, preferably in an amount in the range of from about 0.5% by weight to about 1 % by weight, or any amount or range therein, more preferably in an amount of about 0.75% by weight.

DISSOLUTION: In an embodiment of the present invention, the immediate release layer exhibits a dissolution rate (as measured by USP apparatus I, 200 rpm rotation speed, 900 mL 0.5% POLYSORBATE 20 in water) of greater than or equal to about 60% of the compound of formula (I-X) release within 45 minutes, preferably greater than or equal to about 75% of the compound of formula (I-X) released within 45 minutes, more preferably greater than or equal to about 90% of the compound of formula (I-X) release within 45 minutes, more preferably greater than or equal to about 98% of the compound of formula (I-X) release within 45 minutes.

### EXTENDED RELEASE LAYER:

In an embodiment of the present invention, the extended release layer comprises an internal phase granule comprising metformin hydrochloride and one or more suitable pharmaceutically acceptable excipients (preferably a binder); and an extra-granular phase comprising one or more suitable pharmaceutically acceptable excipients (and preferably containing no metformin hydrochloride). In another embodiment, the extended release layer comprises a compression mixture, which compression mixture is the product of the admixture of the internal phase granule and the extra-granular phase.

In an embodiment of the present invention, the extended release layer comprises metformin hydrochloride and one or more suitable pharmaceutically acceptable excipients. In another embodiment, the extended release layer comprises an internal phase granule comprising metformin HCl; wherein the internal phase granule is admixed with one or more suitable excipients (as part of an extra-granular phase) to yield a compression mixture. In another embodiment of the present invention, the internal phase granule further comprises one or more binders. In another embodiment of the present invention, the internal phase granule comprises metformin HCl and hydroxypropylmethylcellulose. In another embodiment of the present invention, extra-granular phase comprises one or more of the following pharmaceutically acceptable excipients: (a) one or more binders, (b) one or more control release excipients, (c) one or more fillers, (d) one of more flow regulators (or glidants) and / (e) one or more lubricants. Preferably, the extra-granular phase comprises one or more control release excipients.

INTERNAL PHASE GRANULE: In an embodiment of the present invention, the internal phase granule comprises metformin hydrochloride and one or more binders, preferably hydroxypropylmethylcellulose, and optionally one or more fillers. Preferably, the binder is present in an amount in the range of from about 1% to about 10% by weight, or any amount or range therein, (relative to the weight of metformin hydrochloride present in the internal phase granule), preferably, in an amount in the range of from about 1% to about 4% by weight, or any amount or range therein, more preferably in an amount in the range of from about 1.5% to about 2% by weight, or any amount or range therein.

EXTRA-GRANULAR PHASE: In an embodiment of the present invention, the extra-granular phase comprises one or more of the following components / excipient: (a) one or more control release excipients, (b) one or more binders, (c) one or more fillers, (d) one or more flow regulators and (e) one or more lubricants. In another embodiment of the present invention, the extra-granular phase is present in an amount in the range of from about 30% to about 75% by weight, or any amount or range therein (relative to the weight of the internal phase granule), preferably in an amount in the range of from about 50% to about 65%, by weight, or any amount or range therein, more preferably in an amount in the range of from about 57% to about 61 % by weight, or any amount or range therein. (One skilled in the art will recognize that an amount of about 57% by weight relative to the weight of the internal phase granule corresponds to an amount of about 36% by weight relative to the total weight of the extended release layer). In an embodiment of the present invention, the extra-granular phase is present in a ratio relative to the internal phase granule in the range of from about 12:1 to about 1:6, or any amount or range therein, preferably in a ratio of from about 5:1 to about 1:5, or any amount or range therein, more preferably in a ratio in the range of from about 2.5:1 to about 1:2.5, or any amount or range therein.

CONTROL RELEASE EXCIPIENT: In an embodiment of the present invention, the one or more control release excipients (in the extra-granular phase of the extended release layer) is one or more polymers (wherein the polymers include, but are not limited to carbopolymers and hypomellose, and the like), preferably one or more carbomers. Preferably, one or more the control release excipients are a mixture of carbopolymers and hypromellose. In another embodiment, the one or more control release excipients are present in an amount in the range of from about 10% to about 35% by weight (relative to the weight of the total weight of the (extended release compression mixture), or any amount or range therein, preferably in an amount in the range of from about 15% to about 28% by weight, or any amount or range therein, more preferably in an amount of about 28% by weight.

In another embodiment of the present invention, the one or more control release excipients in the extra-granular phase are a mixture of two carbopolymers, wherein the two carbopolymer are present in about equal amount (i.e. as a 50/50 w/w mixture). In another embodiment of the present invention, the one or more control release excipients in the extra-granular phase mixture are a mixture of two carbopolymers, wherein the two carbopolymers are present in a w/w ratio or about 3:1. In another embodiment of the present invention, the one or more control release excipients in the extra-granular phase are a mixture of two carbopolymers, and a high molecular weight hydroxypropylmethylcellulose (HPMC). In another embodiment of the present invention, the control release excipients in the extra-granular phase are a mixture of two carbopolymers, wherein the carbopolymers are present in a ratio of about 1:1 and high molecular weight HPMC. In another embodiment of the present invention, the ratio of HPMC to the one or more carbopolymers is in the range of from about 1:1 to about 3:1, or any amount or range therein, preferably, the ratio is in the range of from about 1.6:1 to about 2.5:1, or any amount or range therein, more preferably, the ratio is about 1.9:1.

HPMC IN EXTRA-GRANULAR PHASE: In an embodiment of the present invention, the extra-granular phase further comprises hydroxypropylmethylcellulose (HPMC). In another embodiment, the HPMC in the extra-granular phase is present in an amount in the range of from about 25% to about 75% by weight (relative to the weight of the extra-granular phase), or any amount or range therein, more preferably in an amount in the range of from about 45% to about 65% by weight, or any amount or range therein, more preferably in an amount of about 55% by weight. In another embodiment of the present invention, the hydropropylmethylcellulose is a high molecular weight hydroxypropylmethylcellulose and is present in an amount in the range of from about 20% to about 30% by weight, or any amount or range therein, preferably in an amount of about 25% by weight.

In an embodiment of the present invention, the extra-granular phase comprises a mixture of linear and reticular polymers. In another embodiment of the present invention, the linear polymer is HPMC, preferably a high molecular weight HPMC. In another embodiment of the present invention, the reticulated polymer is one or more carbopolymers. Preferably, the one or more carbopolymers is a mixture of two carbopolymers.

FILLER: In an embodiment of the present invention, the filler (in the extra-granular phase) is silicified microcrystalline cellulose, microcrystalline cellulose or a mixture thereof. In another embodiment, the filler is present in an amount in the range of from about 10% to about 40% by weight (relative to the weight of the extra-granular phase), or any amount or range therein, more preferably in an amount in the range of from about 15% to about 25% by weight, or any amount or range therein, more preferably in an amount of about 20.5% by weight. In another embodiment of the present invention, the filler (in the extra-granular phase) is a mixture of silicified microcrystalline cellulose and microcrystalline cellulose and is present in an amount in the range of from about 50% to about 75% by weight, or any amount or range therein, preferably in an amount in the range of from about 55% to about 65% by weight, or any amount or range therein, more preferably in an amount of about 61 %.

FLOW REGULATOR (or GLIDANT): In an embodiment of the present invention, the flow regulator (in the extra-granular phase) is colloidal anhydrous silica. In another embodiment, the flow regulator is present in an amount in the range of from about 0.1 % to about 2.5% by weight (relative to the weight of the extra-granular phase), or any amount or range therein, more preferably in an amount in the range of from about 0.5% to about 1.5% by weight, or any amount or range therein, more preferably in an amount in the range of from about 0.8% to about 1.1 % by weight, or any amount or range therein.

LUBRICANT: In an embodiment of the present invention, the lubricant (in the extra-granular phase) is magnesium stearate. In another embodiment of the present invention, the lubricant is present in an amount in the range of from about 0.1 % by weight to about 3% by weight (relative to the total weight of the extra-granular phase), or any amount or range therein, preferably in an amount in the range of from about 0.4% by weight to about 2% by weight, or any amount or range therein, more preferably in an amount in the range of from about 0.8% to about 1.1 % by weight, or any amount or range therein.

DISSOLUTION: In an embodiment of the present invention, the extended release layer exhibits a dissolution rate, as measured by 1,000 mL 0.05M phosphate buffer pH 6.8, Apparatus II (Paddle) at 100 rpm using a sinker (corresponding to USP method test n°8), of from about 25% to about 45% of the metformin released after about 1 hour; from about 50% to about 70% of the metformin released after about 3 hours; and at least 80% of the metformin released after about 10 hours.

In an certain preferred embodiments of the present invention, the extended release layer exhibits a dissolution rate, as measured by 1,000 mL 0.05M phosphate buffer pH 6.8, Apparatus II (Paddle) at 100 rpm using a sinker (corresponding to USP method test n°8), of from about 20% to about 40% of the metformin released after about 1 hour; from about 30% to about 50% of the metformin released after about 2 hours, from about 65% to about 85% of the metformin release after about 6 hours, and at least 85% of the metformin released after about 10 hours.

In an embodiment of the present invention, the immediate release layer comprises (a) the compound of formula (I-X) in a crystalline hemihydrate form, in an amount of about 153 mg; (b) microcrystalline cellulose, in amount of about 59 mg; (c) lactose anhydrate in an amount of about 59 mg; (d) croscamellose sodium in an amount of about 18 mg; (e) hydroxypropylcellulose in an amount of about 9 mg; and (f) magnesium stearate in an amount of about 2.2 mg.

In an embodiment of the present invention, the immediate release layer comprises (a) the compound of formula (I-X) in a crystalline hemihydrate form, in an amount of about 51 mg; (b) microcrystalline cellulose, in amount of about 20 mg; (c) lactose anhydrate in an amount of about 20 mg; (d) croscamellose sodium in an amount of about 6 mg; (e) hydroxypropylcellulose in an amount of about 3 mg; and (f) magnesium stearate in an amount of about 0.74 mg.

In another embodiment of the present invention, the extended release layer comprises (a) an internal phase granule comprising metformin HCl in an amount of about 500 mg and hydroxypropylmethylcellulose 5mPa.s in an amount of about 7.5 mg; and (b) an extra-granular phase comprising CARBOMER 971 P in an amount of about 78 mg; CARBOMER 71 G in an amount of about 26 mg; and hydroxypropylmethylcellulose 100,000 mPa.s in an amount of about 195 mg.

In another embodiment of the present invention, the extended release layer comprises (a) an internal phase granule comprising metformin HCl in an amount of about 500 mg and hydroxypropylmethylcellulose 5mPa.s in an amount of about 7.5 mg; and (b) an extra-granular phase comprising CARBOMER 971 P in an amount of about 78 mg; CARBOMER 71 G in an amount of about 26 mg; hydroxypropylmethylcellulose 100,000 mPa.s in an amount of about 195 mg; silicified microcrystalline cellulose in an amount of about448 mg; microcrystalline cellulose in an amount of about 32.5 mg; colloidal anhydrous silica in an amount of about 6.5 mg; and magnesium stearate in an amount of about 6.5 mg.

### PREPARATION

The present invention is further directed to processes for the preparation of the pharmaceutical compositions as described herein, preferably to processes for the preparation of bi-layer tablet compositions as described herein. In an embodiment, the present invention is directed to the preparation of a bi-layer tablet; wherein the bi-layer tablet comprises (a) an extended release layer comprising metformin hydrochloride and (b) an immediate release layer comprising a compound of formula (I-X) or pharmaceutically acceptable salt thereof, preferably a crystalline hemihydrate form of the compound of formula (I-X).

In an embodiment of the present invention, the present invention is directed to a process for the preparation of a bi-layer tablet as described herein comprising (a) preparing a compression mixture for the extended release layer (comprising metformin HCl) and compressing said compression mixture to form a tablet layer; (b) preparing granules of the immediate release layer (comprising the compound of formula (I-X)) and compressing said granules together with the pre-formed tablet layer comprising the metformin HCl to form a bi-layer tablet. Preferably, the bi-layer tablet is coated according to known methods.

In another embodiment, the present invention is directed to a process for the preparation of a bi-layer tablet comprising
(a) preparing an internal phase granule comprising metformin hydrochloride and a low molecular weight hydroxypropylmethylcellulose;
(b) admixing the internal phase granule with one or more carbopolymers and a hydroxypropylmethylcellulose to yield a compression mixture;
(c) compressing the compression mixture to yield a first tablet layer;
(d) preparing a granule of a compound of formula (I-X) or pharmaceutically acceptable salt thereof and one or more excipients;
(e) compressing the granule with the first tablet layer; to yield a bi-layer tablet; and
(f) optionally coating the bi-layer tablet.

In an embodiment of the present invention, the immediate release layer is prepared as granules of the desired components, more particularly by mixing the compound of formula (I-X) or pharmaceutically acceptable salt thereof with one or more pharmaceutically acceptable excipients, for example with one or more fillers, one or more disintegrants, one or more binders and / or one or more lubricants, to yield granules; which granules are optionally screened through a suitably selected mesh screen. The granules are then preferably pressed, according to known methods, to form a tablet form layer.

In an embodiment of the present invention, the extended release layer is prepared from a compression mixture, wherein the compression mixture is prepared by admixing an internal phase granule with and extra-granular phase. In another embodiment of the present invention, the internal phase granule contains the active ingredient, preferably metformin HCl. In another embodiment of the present invention, the extra-granular phase contains one or more excipients which provide the extended release characteristics of the extended release layer.

In an embodiment of the present invention, the extended release layer is prepared according to the following steps:
STEP A: admixing metformin hydrochloride and optionally, a binder, according to known methods, to yield an internal phase granule;
STEP B: optionally screening said internal phase granule through a suitably selected mesh screen;
STEP C: admixing extra-granular components (preferably, one or more control release excipients, one or more fillers, and / or one of more flow regulators) and the internal phase granule prepared in STEP A, to form a non-lubricated mixture;
STEP D: admixing the lubricant to the non-lubricated mixture to yield a compression mixture; and
STEP E: compressing the compression mixture to form a tablet layer.

TABLET SIZE: In an embodiment of the present invention, the pharmaceutical composition (preferably solid oral dosage form, more preferably, bi-layer tablet comprising (a) an extended release layer comprising metformin hydrochloride and (b) immediate release layer comprising a compound of formula (I-X) or pharmaceutically acceptable salt thereof is present in a total weight of less than about 2,000 mg, such that it may be readily swallowed by a patient. Preferably, the tablet is present in a total weight in the range of from about 500 mg to about 2000 mg, or any amount or range therein, more preferably, in a total weight in the range of from about 800 mg to about 2000 mg, or any amount or range therein.

The immediate and extended release layers of the pharmaceutical compositions of the present invention further may be prepared according to known methods and employing known processes and equipment, as disclosed, for example in Pharmaceutical Sciences, Remington, 17th Ed., pp. 1585-1594 (1985); Chemical Engineers Handbook, Perry, 6th Ed., pp. 21-13 to 21-19 (1984); Journal of Pharmaceutical Sciences, Parrot, Vol. 61, No. 6, pp. 813-829 (1974); and Chemical Engineer, Hixon, pp. 94-103 (1990).

Manufacturing the granules / particles for the immediate release of the pharmaceutical compositions of the present invention may be performed, for example, by comminution, which produces the desired size of the active ingredient and the desired size of any accompanying pharmaceutically acceptable excipient(s). Suitable means for producing the desired particles include, but are not limited to, granulation, spray drying, sieving, lyophilization, crushing, grinding, jet milling, micronizing and chopping to produce the intended particle size. The process can be performed by size reduction equipment, such as a micropulverizer mill, a fluid energy-grinding mill, a grinding mill, a roller mill, a hammer mill, an attrition mill, a chaser mill, a ball mill, a vibrating ball mill, an impact pulverizer mill, a centrifugal pulverizer, a coarse crusher and a fine crusher. The size of the particle can be ascertained by screening, including a grizzly screen, a flat screen, a vibrating screen, a revolving screen, a shaking screen, an oscillating screen and a reciprocating screen.

In an embodiment, the immediate release of the pharmaceutical compositions of the present invention may be manufactured according to, for example, the wet granulation technique. In the wet granulation technique, solid particles are wetted and bound together by a binder solution consisting essentially of a granulation solvent, generally a binder, and optionally other ingredients. Generally the drug or active ingredient (for example, the compound of formula (I-X) or pharmaceutically acceptable salt thereof) is granulated as solid particles together with (or without) solid excipients, or is partially dissolved in the binder solution. The solid particles can be mixed by means of mechanical agitation (low or high shear mixer) or fluidized by a gas (as in fluid bed granulation). The granulating fluid is added until a wet blend is produced, which wet mass blend is then forced through a predetermined screen and dried in a fluid bed dryer. The blend is dried for about 18 to about 24 hours at a temperature in the range of from about 24°C to about 35°C in a forced-air oven. The dried granules are then sized, according to known methods. The dried granules are then sized. Next, magnesium stearate, or another suitable lubricant (if desired) and other excipient materials (as appropriate) are added to the granulation, and the granulation is put into milling jar sand mixed on a jar mill for 10 minutes. The resulting composition is pressed into a layer, for example, in a Manesty^{®} press or a Korsch LCT press. In an example, the speed of the press is set at 15 rpm and the maximum load set at about 4 tons.

In another embodiment, the active ingredient and other pharmaceutically acceptable excipients comprising either the immediate release or extended release layer of the composition of the present invention may be blended and pressed into a solid layer. The layer possesses dimensions that correspond to the internal dimensions of the area the layer is to occupy in the dosage form. The active ingredient and other pharmaceutically acceptable excipients can also be blended with a solvent and mixed into a solid or semisolid form by conventional methods, such as ballmilling, calendering, stirring or rollmilling, and then pressed into a preselected shape.

In another embodiment of the present invention, the manufacturing process comprises blending the powdered ingredients (active ingredient and other pharmaceutically acceptable excipient(s)) in a fluid bed granulator. After the powdered ingredients are dry blended in the granulator, a granulating fluid, for example, polyvinylpyrrolidone in water, is sprayed onto the powders, which provokes the agglomeration of the particles together. The agglomerated materials are then dried in the granulator. This process granulates all the ingredients present therein while adding the granulating fluid. After the granules are dried, a lubricant, such as stearic acid or magnesium stearate, is mixed into the granulation using a blender e.g., V-blender or tote blender. The granules are then pressed and coated in the manner described above.

Exemplary solvents suitable for manufacturing the pharmaceutical composition components comprise aqueous or inert organic solvents that do not adversely harm the materials used in the system. The solvents broadly include members selected from the group consisting of aqueous solvents, alcohols, ketones, esters, ethers, aliphatic hydrocarbons, halogenated solvents, cycloaliphatics, aromatics, heterocyclic solvents and mixtures thereof. Typical solvents include acetone, diacetone alcohol, methanol, ethanol, isopropyl alcohol, butyl alcohol, methyl acetate, ethylacetate, isopropyl acetate, n-butyl acetate, methyl isobutyl ketone, methyl propyl ketone, n-hexane, n-heptane, ethylene glycol monoethyl ether, ethylene glycol monoethyl acetate, methylene dichloride, ethylene dichloride, propylene dichloride, carbon tetrachloridenitroethane, nitropropane tetrachloroethane, ethyl ether, isopropyl ether, cyclohexane, cyclooctane, benzene, toluene, naphtha, 1,4-dioxane, tetrahydrofuran, diglyme, water, aqueous solvents containing inorganic salts such as sodium chloride, calcium chloride, and the like, and mixtures thereof such as acetone and water, acetone and methanol, acetone and ethyl alcohol,methylene dichloride and methanol, and ethylene dichloride and methanol.

Exemplary liquid carriers for the present invention include surfactants, and hydrophilic solvents. Exemplary surfactants for example, include, but are not limited to, Vitamin E TPGS, Cremophor® (grades EL, EL-P, and RH40), Labrasol®, Tween® (grades 20, 60, 80), Pluronic® (gradesL-31, L-35, L-42, L-64, and L-121), Acconon® S-35, Solutol HS-15, and Span (grades 20, and 80). Exemplary hydrophilic solvents for example, include, but are not limited to, Isosorbide Dimethyl Ether, Polyethylene Glycol (PEG grades 300, 400, 600, 3000, 4000, 6000, and 8000) and Propylene Glycol (PG).

### BILAYER TABLET FORMATION:

Shaping into tablets is generally performed from the compression of particulate solids. This solid form may be obtained by blending, milling, spray drying, dry- wet- or melt-granulating or a combination thereof. In other cases the tablets may be formed by molding (e.g injection molding), by solidification by evaporation of solvent from solution disposed in molds, wherein those cases the product is usually formed when hot and allowed to solidify on cooling. The shaped product may likewise be produced in film or sheet form by evaporation or by pouring a heated mass onto a plate and evaporating off the solvent.

For a bi-layered tablet, granules or powders of the first layer (e.g. the extended release layer) and the second layer (e.g. the immediate release layer) are sequentially placed in an appropriately-sized die with intermediate compression step being applied to the first layer, followed by a final compression step after the second layer is added to the die to form the bilayered core. The intermediate compression typically takes place under a pressure of no more than a few hundred kg/cm². Final stage compression typically takes place at typical compression forces, which are dependent on the composition and size of the compact.

Where desired, pan coating may be conveniently used to provide the completed dosage form. In the pan coating system, the coating composition is deposited by successive spraying onto the compressed tablet, accompanied by tumbling in a rotating pan. A pan coater is commonly used because of its availability at commercial scale. Other techniques can be used for coating the tablet. Once coated, the tablet is dried in, for example, in the same coating pan equipment, or in a forced-air oven or in a temperature and humidity controlled oven to free the dosage form of solvent(s) used in the manufacturing. Drying conditions are conventionally chosen on the basis of available equipment, ambient conditions, solvents, coatings, coating thickness, and the like.

Other coating techniques can also be employed. For example, one alternative technique uses an air-suspension procedure. This procedure consists of suspending and tumbling the tablet in a current of air, until a coating is applied. The air-suspension procedure is described in, for example, U.S. Patent No. 2,799,241; in J. Am. Pharm. Assoc., Vol. 48, pp. 451-459 (1959); and, ibid., Vol. 49, pp. 82-84 (1960). The tablet also can be coated with a Wurster® air-suspension coater using, for example, methylene dichloride methanol as a co-solvent for the coating material. An Aeromatic® air-suspension coater can be used employing a co-solvent.

Tablets may be further printed for improved identification, or waxed, for esthetical reasons.

As used herein, unless otherwise noted, the term **"immediate-release"** shall refer to release of at least about 75% (preferably at least about 80%, more preferably at least about 90%, more preferably at least about 95%, more preferably at least about 98%) of the active ingredient of the pharmaceutical composition or layer within a short time period following administration, preferably within less than about 1 hour, more preferably, within about 45 minutes.

In certain embodiment, the present invention is directed to bi-layer tablet compositions comprising (a) and an extended release layer comprising metformin hydrochloride; and (b) an immediate release layer comprising a compound of formula (I-X) or pharmaceutically acceptable salt thereof (preferably a crystalline hemihydate form the compound of formula (I-X)); wherein at least about 75% of the compound of formula (I-X) is released from the bi-layer tablet within about 45 min of administration. Preferably at least about 90% of the compound of formula (I-X) is released from the bi-layer tablet within about 45 min of administration.

As used herein, unless otherwise noted, the term **"extended release"** shall refer to release of the active ingredient of the pharmaceutical composition or layer substantially continuously for at least about 4 hours, preferably for at least about 12 hours, more preferably from about 5 to about 24 hours. In an embodiment, extended release compositions and / or layers of the present invention exhibit T₇₀ values (i.e. time to release of about 70% of the active ingredient) in the range of from about 4 hours to about 24 hours, or any amount or range therein, preferably, in the range of from about 5 hours to about 24 hours, or any amount or range therein. In an embodiment of the present invention, the release of the active ingredient of the pharmaceutical composition or layer is substantially continuous for from about 5 hours to about 16 hours, or any amount or range therein.

In certain embodiments, the present invention is directed to bi-layer tablet compositions comprising an immediate release layer comprising a compound of formula (I-X) or pharmaceutically acceptable salt thereof (preferably, a crystalline hemihydrate form of the compound of formula (I-X)) and an extended release layer comprising metformin hydrochloride, wherein at least about 85% of the metformin hydrochloride is released within about 10 hours of administration.

In certain embodiments, the present invention is directed to bi-layer tablet compositions comprising an immediate release layer comprising a compound of formula (I-X) or pharmaceutically acceptable salt thereof (preferably, a crystalline hemihydrate form of the compound of formula (I-X)) and an extended release layer comprising metformin HCl, wherein between about 25% and about 45% of the metformin HCl is release within about 1 hour of administration; wherein between about 50% and about 70% of the metformin HCl is released within about 3 hours of administration; and wherein at least 80% of the metformin HCl is release within about 10 hours of administration.

In certain embodiments, the present invention is directed to bi-layer tablet compositions comprising an immediate release layer comprising a compound of formula (I-X) or pharmaceutically acceptable salt thereof (preferably, a crystalline hemihydrate form of the compound of formula (I-X)) and an extended release layer comprising metformin HCl, wherein between about 30% and about 50% of the metformin HCl is release within about 1 hour of administration; wherein between about 60% and about 80% of the metformin HCl is released within about 3 hours of administration; and wherein at least 85% (more preferably at least about 90%) of the metformin HCl is release within about 10 hours of administration.

As used herein, unless otherwise noted, the term **"substantially uniform release rate"** shall mean an average hourly release rate that varies positively or negatively by no more than about 30%, preferably by no more than about 25%, more preferably, by no more than 10% from either the preceding or the subsequent average hourly release rate, as determined according to known methods.

In an embodiment of the present invention, the immediate release layer of pharmaceutical compositions of the present invention release the compound of formula (I-X) with a substantially uniform release rate. In another embodiment of the present invention, the extended release layer of the pharmaceutical compositions of the present invention release the metformin HCl with a substantially uniform release rate.

### METHODS OF TREATMENT

The present invention is further directed to compositions for use in methods for the treatment and prevention of (preferably, the prevention of the development of) glucose related disorders comprising administering to a subject in need thereof a therapeutically effective amount of any of the pharmaceutical compositions as described herein, preferably the bi-layer tablets comprising (a) an extended release layer comprising metformin or a pharmaceutically acceptable salt thereof, preferably metformin hydrochloride; and (b) an immediate release layer comprising a compound of formula (I-X) or pharmaceutically acceptable salt thereof, preferably the crystalline hemihydrate form of the compound of formula (I-X).

The methods according to the present inventions are directed to the treatment and or prevention (including delay in the progression or onset of) of "glucose-related disorders". As used herein, the term **"glucose related disorder"** shall be defined as any disorder which is characterized by or is developed as a consequence of elevated glucose levels. Glucose-related disorders shall include diabetes mellitus, diabetic retinopathy, diabetic neuropathy, diabetic nephropathy, delayed wound healing, insulin resistance, hyperglycemia, hyperinsulinemia, elevated blood levels of fatty acids, elevated blood levels of glucose, postprandial hyperglycemia, hyperlipidemia, obesity, hypertriglyceridemia, Syndrome X, diabetic complications, atherosclerosis, hypertension, hypercholesterolemia, mixed dyslipidemia, fatty liver, and/or nonalcoholic fatty liver disease. In particular, the "glucose related-disorder" is diabetes mellitus (type 1 and type 2 diabetes mellitus, etc.), diabetic complications (such as diabetic retinopathy, diabetic neuropathy, diabetic nephropathy), obesity, or postprandial hyperglycemia.

In an embodiment of the present invention, the glucose related disorder is selected from the group consisting of diabetes mellitus, diabetic retinopathy, diabetic neuropathy, diabetic nephropathy, delayed wound healing, insulin resistance, hyperglycemia, hyperinsulinemia, elevated blood levels of fatty acids, hyperlipidemia, obesity, hypertriglyceridemia, Syndrome X, diabetic complications, atherosclerosis and hypertension.

In another embodiment of the present invention, glucose related disorder is selected from the group consisting of type 1 diabetes mellitus, type 2 diabetes mellitus, diabetic retinopathy, diabetic neuropathy, diabetic nephropathy, obesity and postprandial hyperglycemia. In another embodiment of the present invention, the glucose related disorder is selected from the group consisting of type 1 diabetes mellitus, type 2 diabetes mellitus, diabetic retinopathy, diabetic neuropathy, diabetic nephropathy, obesity, and delayed wound healing. In another embodiment of the present invention, the glucose related disorders is selected from the group consisting of poor glycemic control, Type 2 Diabetes Mellitus, Syndrome X, gestational diabetes, insulin resistance, hyperglycemia. In another embodiment of the present invention, the glucose related disorder is Type 2 diabetes mellitus.

In another embodiment, the glucose related disorder is selected from the group consisting of elevated glucose level, pre-diabetes, impaired oral glucose tolerance, poor glycemic control, Type 2 Diabetes Mellitus, Syndrome X (also known as metabolic syndrome), gestational diabetes, insulin resistance, and hyperglycemia.

Treatment of glucose related disorders may comprise lowering glucose levels, improving glycemic control, decreasing insulin resistance and / or preventing the development of a glucose related disorder (for example preventing a patient suffering from impaired oral glucose tolerance or elevated glucose levels from developing Type 2 diabetes mellitus).

As used herein, the terms **"Syndrome X", "Metabolic Syndrome"** and **"Metabolic Syndrome X"** shall mean a disorder that presents risk factors for the development of Type 2 diabetes mellitus and cardiovascular disease and is characterized by insulin resistance and hyperinsulinemia and may be accompanied by one or more of the following: (a) glucose intolerance, (b)Type 2 diabetes mellitus, (c) dyslipidemia, (d) hypertension and (e) obesity.

The term **"subject"** as used herein, refers to an animal, preferably a mammal, most preferably a human, who has been the object of treatment, observation or experiment.

As used herein, unless otherwise noted, the terms **"treating", "treatment"** and the like, shall include the management and care of a subject or patient (preferably mammal, more preferably human) for the purpose of combating a disease, condition, or disorder and includes the administration of a compound of the present invention to prevent the onset of the symptoms or complications, alleviate the symptoms or complications, or eliminate the disease, condition, or disorder.

As used herein, unless otherwise noted, the term **"prevention"** shall include (a) reduction in the frequency of one or more symptoms; (b) reduction in the severity of one or more symptoms; (c) the delay or avoidance of the development of additional symptoms; and / or (d) delay or avoidance of the development of the disorder or condition.

One skilled in the art will recognize that wherein the present invention is directed to methods of prevention, a subject in need of thereof (i.e. a subject in need of prevention) shall include any subject or patient (preferably a mammal, more preferably a human) who has experienced or exhibited at least one symptom of the disorder, disease or condition to be prevented. Further, a subject in need thereof may additionally be a subject (preferably a mammal, more preferably a human) who has not exhibited any symptoms of the disorder, disease or condition to be prevented, but who has been deemed by a physician, clinician or other medical profession to be at risk of developing said disorder, disease or condition. For example, the subject may be deemed at risk of developing a disorder, disease or condition (and therefore in need of prevention or preventive treatment) as a consequence of the subject's medical history, including, but not limited to, family history, pre-disposition, co-existing (comorbid) disorders or conditions, genetic testing, and the like.

The term **"therapeutically effective amount"** as used herein, means that amount of active compound or pharmaceutical agent that elicits the biological or medicinal response in a tissue system, animal or human that is being sought by a researcher, veterinarian, medical doctor or other clinician, which includes alleviation of the symptoms of the disease or disorder being treated.

Wherein the present invention is directed to co-therapy or combination therapy, comprising administration of (a) metformin or a pharmaceutically acceptable salt thereof and (b) a compound of formula (I-X) or a pharmaceutically acceptable salt thereof, **"therapeutically effective amount"** shall mean that amount of the combination of agents taken together so that the combined effect elicits the desired biological or medicinal response. For example, the therapeutically effective amount of co-therapy comprising administration of (a) metformin or a pharmaceutically acceptable salt thereof and (b) a compound of formula (I-X) or a pharmaceutically acceptable salt thereof, would be the amount of (a) the metformin or a pharmaceutically acceptable salt thereof and (b) the compound of formula (I-X) or pharmaceutically acceptable salt thereof that when taken together or sequentially have a combined effect that is therapeutically effective. Further, it will be recognized by one skilled in the art that in the case of co-therapy with a therapeutically effective amount, as in the example above, the amount of the (a) metformin or pharmaceutically acceptable salt thereof and / or the amount of the (b) compound of formula (I-X) or pharmaceutically acceptable salt thereof individually may or may not be therapeutically effective.

Optimal dosages to be administered may be readily determined by those skilled in the art, and will vary with for example, the mode of administration, the strength of the preparation, the mode of administration, and the advancement of the disease condition. In addition, factors associated with the particular patient being treated, including patient age, weight, diet and time of administration, will result in the need to adjust dosages.

One skilled in the art will recognize that, both *in vivo* and *in vitro* trials using suitable, known and generally accepted cell and / or animal models are predictive of the ability of a test compound or co-therapy to treat or prevent a given disorder. One skilled in the art will further recognize that human clinical trials including first-in-human, dose ranging and efficacy trials, in healthy patients and / or those suffering from a given disorder, may be completed according to methods well known in the clinical and medical arts.

To provide a more concise description, some of the quantitative expressions herein are recited as a range from about amount X to about amount Y. It is understood that wherein a range is recited, the range is not limited to the recited upper and lower bounds, but rather includes the full range from about amount X through about amount Y, or any amount or range therein.

To provide a more concise description, some of the quantitative expressions given herein are not qualified with the term **"about".** It is understood that whether the term "about" is used explicitly or not, every quantity given herein is meant to refer to the actual given value, and it is also meant to refer to the approximation to such given value that would reasonably be inferred based on the ordinary skill in the art, including approximations due to the experimental and/or measurement conditions for such given value.

As used herein, the term **"composition"** is intended to encompass a product comprising the specified ingredients in the specified amounts, as well as any product which results, directly or indirectly, from combinations of the specified ingredients in the specified amounts.

The following Examples are set forth to aid in the understanding of the invention, and are not intended and should not be construed to limit in any way the invention set forth in the claims which follow thereafter.

Examples 1-4 which follow herein describe pharmaceutical composition. Unless otherwise noted, wherein a prepared pharmaceutical composition was measured to determine the dissolution profile of metformin HCl within the composition, said dissolution profile was measured according to standard USP procedures, using Apparatus II. Similarly, unless otherwise noted, wherein a prepared pharmaceutical composition was measured to determine the dissolution profile of the compound of formula (I-X), said dissolution profile was measured according to standard USP procedures, using Apparatus I.

### Example 1 (Reference)

### Mono-Layer and Bi-Layer Tablet Pharmaceutical Composition

### Mono-Layer Pharmaceutical Composition / Tablet

Five mono-layer extended release tablet compositions comprising metformin HCl were prepared from a combined internal phase granule and an extra-granular phase, as described in more detail below. Table 1A below, provides a listing of the components and amounts of said components present.

**Table 1A: Mono-Layer, Extended Release Tablet Composition**

| **Component** | **TAB 6-1** | **TAB 6-3** | **TAB 6-4** | **TAB 6-5** | **TAB 6-6** |
|---|---|---|---|---|---|
| **Internal phase granule** | | | | | |
| Metformin HCl | 1000 mg | 1000 mg | 1000 mg | 1000 mg | 1000 mg |
| Hydroxypropyl Methylcellulose (HPMC 2910 5mPa.s) | | 20 mg | 20 mg | 20 mg | 20 mg |
| Carbomer 971 P NF polymer | 195 mg | | | | |

| **Extra-Granular Phase** | | | | | |
|---|---|---|---|---|---|
| Carbomer 971 P | | 128 mg | 160 mg | 128 mg | 128 mg |
| Hydroxypropyl Methylcellulose (HPMC 2208 100,000mPa.s) | 200 mg | 320 mg | 256 mg | 256 mg | 200 mg |
| Silicified Microcrystalline Cellulose | 157 mg | 84 mg | 116 mg | 148 mg | 204 mg |
| Microcrystalline Cellulose | 35 mg | 35 mg | 35 mg | 35 mg | 35 mg |
| Colloidal Anhydrous Silica | 6.5 mg | 6.5 mg | 6.5 mg | 6.5 mg | 6.5 mg |
| Magnesium stearate | 6.5 mg | 6.5 mg | 6.5 mg | 6.5 mg | 6.5 mg |

### Extended Release Compression Mixture Preparation:

Metformin hydrochloride (purchased from Granules India) with a mean particle size (d50) of 198µm (as determined by laser diffraction) / 74.9% w/w retained on 200mesh screen (supplier information) was screened through a 0.95mm sieve and loaded in a Glatt GPCG1 or Glatt GPCG30 fluid bed granulator (Glatt). The metformin HCl was then granulated with an aqueous solution of the hydropropylmethylcellulose (HPMC 2910 15mPa.s; binder concentration of 5.66% w/w solids) sprayed through a 1 mm (for TAB-6-1 and TAB-6-2) or 1.8 mm nozzle (for TAB-6-3, TAB-6-4, TAB-6-5 and TAB-6-6); for formulation TAB-6-1 the carbopolymer 971 P was added to the metformin HCl in the granulator, and the powders were granulated with purified water. Inlet air, outlet air and product bed temperatures were monitored throughout the process. The granules thus obtained were dried in the same equipment. When the Glatt GPGC1 fluid bed granulator was used, the drying was to a target moisture content of 0.5% w/w (by Loss on drying); whereas when the Glatt GPGC30 fluid bed granulator was used, the drying was to a target moisture content of about 0.1-0.2% w/w (by Loss on drying). The granules were allowed to cool down and were then sieved through a 0.95mm sieve.

The resulting granules and extra-granulate phase components were weighed separately and sieved together through a 0.95mm sieve. The sieved materials were blended in a Turbula mixer for 5 min or in a bin blender for 10 min. to yield the extended release compression mixture.

### Tablet Formation:

Tablet manufacturing was performed on a single punch tablet press (Courtoy) equipped with oblong punches. The tablet was prepared by compressing the above prepared extended release compression mixture.

### Bi-layer Pharmaceutical Composition / Tablet

Additionally, a bi-layer tablet composition comprising an immediate release layer containing the equivalent of 150 mg of the compound of formula (I-X) and an extended release layer containing metformin hydrochloride was prepared as described in more detail below. Table 1 B below, provides a listing of the components and amounts for the immediate release and extended release layers.

**Table 1B: TAB-6-2: Bi-layer Tablet Layer Components**

| **Component** | **Amount** |
|---|---|
| **Extended Release Layer - Internal phase granule** | |
| Metformin HCl | 1000 mg |
| Carbomer 971 P NF polymer | 195 mg |

| **Extended Release Layer - Extra-Granular Phase** | |
|---|---|
| Hydroxypropyl Methylcellulose (HPMC 2208 100000 mPa.s) | 280 mg |
| Silicified Microcrystalline Cellulose | 77 mg |
| Microcrystalline Cellulose | 35 mg |
| Colloidal Anhydrous Silica | 6.5 mg |
| Magnesium stearate | 6.5 mg |

| **Immediate Release Layer Granule** | |
|---|---|
| Compound of Formula (I-X) as hemihydrate | 153 mg |
| Microcrystalline cellulose | 58.89 mg |
| Lactose Anhydrous Direct Tableting | 58.89 mg |
| Hydroxypropyl cellulose | 9 mg |
| Croscamellose Sodium (AC-Di-SOL) | 18 mg |
| Magnesium Stearate | 2.22 mg |

### Extended Release Compression Mixture Preparation:

The extended release compression mixture for bi-layer tablet TAB-6-2 was prepared as described above for TAB-6-1.

### Immediate Release Granulate Preparation:

The compound of formula (I-X) as a hemihydrate, microcrystalline cellulose (AVICEL PH 102), anhydrous lactose, and croscarmellose sodium (AC-Di-SOL) were screened through a sieve and loaded in a Glatt GPCG60 fluid bed granulator (Glatt). The powders were granulated with an aqueous solution of hydroxypropyl cellulose (KLUCEL EXF; binder concentration of 5% w/w solids) sprayed through a 1.8mm nozzle. The moisture level was monitored during the process, with samples taken every 10 minutes of the process. A moisture balance was used to determine loss on drying (LOD). The granules thus obtained were dried in the same equipment, to a target moisture content of 1.8% w/w (by loss on drying). The granules were allowed to cool down and were then screened together with the magnesium stearate. The resulting material was blended for 5min in a Bohle mixer.

### Bi-layer Tablet Formation:

Tablet manufacturing was performed on a single punch tablet press (Courtoy)equipped with oblong punches. The first layer compressed was the extended release (metformin HCl containing) layer using the compression mixture prepared as described above. Once the extended release layer was compressed, the immediate release granules (containing the compound of formula (I-X)) prepared as described above were added and the combined material compressed to form the bi-layer tablet.

### Dissolution Characteristics

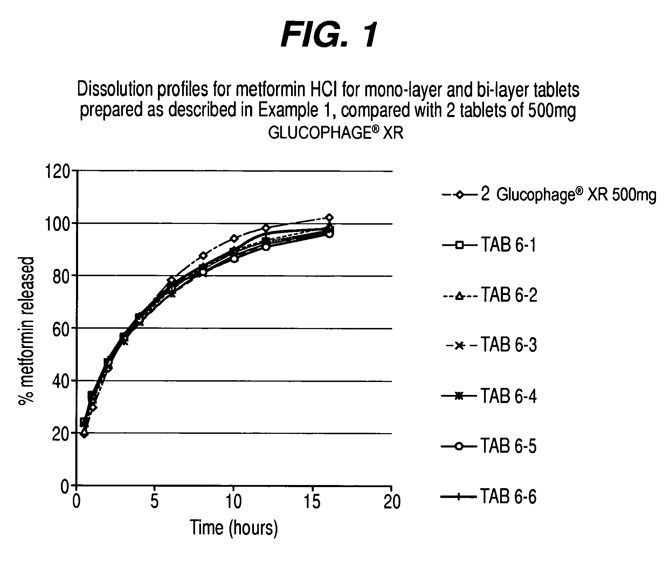

Metformin HCl dissolution profiles for the above prepared tablets (sampling n=3 tablets) were measured using 1,000 mL 0.05M phosphate buffer pH 6.8, Apparatus II (Paddle) at 100 rpm using a sinker (corresponding to USP method test n°8), and compared with the dissolution rate of commercially obtained one or two GLUCOPHAGE^{®} XR 500 mg tablets, as shown in Figure 1. The results, presented as average measured values in Table 1C below, indicate similar average dissolution profiles for the prepared tablets and the GLUCOPHAGE^{®} XR comparator tablets.

**Table 1C: Measured Dissolution (in % Metformin Released)**

| **Time (hours)** | **2 x 500 mg Tablet GLUCOPHAGE^{®} XR** | **TAB-6-1** | **TAB-6-2** | **TAB-6-3** | **TAB-6-4** | **TAB-6-5** | **TAB-6-6** |
|---|---|---|---|---|---|---|---|
| 0.5 | 20 | 24 | 21 | 24 | 24 | 24 | 24 |
| 1 | 30 | 34 | 33 | 33 | 34 | 34 | 35 |
| 2 | 45 | 47 | 46 | 46 | 47 | 47 | 48 |
| 3 | 56 | 56 | 57 | 55 | 55 | 56 | 57 |
| 4 | 64 | 64 | 64 | 62 | 62 | 63 | 65 |
| 6 | 78 | 75 | 75 | 73 | 73 | 75 | 76 |
| 8 | 88 | 83 | 83 | 81 | 81 | 82 | 84 |
| 10 | 94 | 89 | 90 | 87 | 87 | 86 | 90 |
| 12 | 98 | 93 | 94 | 91 | 92 | 91 | 96 |
| 16 | 102 | 97 | 99 | 97 | 97 | 96 | 98 |

### Example 2

### Bi-Layer Tablet Pharmaceutical Compositions

Three bi-layer tablet compositions comprising an immediate release portion containing the equivalent of 150 mg of the compound of formula (I-X) and an extended release portion containing metformin hydrochloride were prepared as described below. The extended release (metformin HCl containing) portion of the tablet further comprised an internal phase granule and an extra-granular phase, which are combined to form the extended release compression mixture. The formulation / layer components and amounts in each said component within the tablet formulation and layers were as listed in Tables 2A and 2B, below.

**Table 2A: Metformin HCl, Extended Release, Layer Components**

| **Component** | **TAB-7-2 & TAB-7-3** | **TAB-7-1** |
|---|---|---|
| **Extended Release Layer - Internal phase granule** | | |
| Metformin HCl | 1000 mg | 500 mg |
| Hydroxypropylmethylcellulose (HPMC 2910 SmPa.s) | 15 mg | 7.5 mg |

| **Extended Release Layer - Extra-Granular Phase** | | |
|---|---|---|
| Carbomer 971 P | 96 mg | 48 mg |
| Carbomer 71 G | 32 mg | 16 mg |
| Hydroxypropylmethylcellulose (HPMC 2208 100,000 mPa.s) | 240 mg | 120 mg |
| Silicified Microcrystalline Cellulose | 161 mg | 80.5 mg |
| Microcrystalline Cellulose | 40 mg | 20 mg |
| Colloidal Anhydrous Silica | 8 mg | 4 mg |
| Magnesium stearate | 8 mg | 4 mg |

**Table 2B: Compound of Formula (I-X), Immediate Release Layer Components**

| **Component** | **Amounts** |
|---|---|
| Compound of Formula (I-X)-as hemihydrate | 153 mg |
| Microcrystalline cellulose (MCC: AVICEL PH 102) | 58.89 mg |
| Lactose Anhydrous Direct Tableting | 58.89 mg |
| Hydroxypropyl cellulose (KLUCEL EXF) | 9 mg |
| Croscamellose Sodium (AC-Di-SOL) | 18 mg |
| Magnesium Stearate (Vegetable) | 2.22 mg |

### Extended Release Granulate Reparation:

Metformin hydrochloride (purchased from Granules India) with a mean particle size (d50) of 198µm (as determined by laser diffraction) / 74.9% w/w retained on 200mesh screen (supplier information) was screened through a 0.95mm sieve and loaded in a Glatt GPCG1 or Glatt GPCG30 fluid bed granulator (Glatt). The metformin HCl was then granulated with an aqueous solution of the hydropropylmethylcellulose (HPMC 2910 15mPa.s; binder concentration of 5.66% w/w solids) sprayed through a 1 mm or 1.8 mm nozzle. Inlet air, outlet air and product bed temperatures were monitored throughout the process. The granules thus obtained were dried in the same equipment. When the Glatt GPGC1 fluid bed granulator was used, the drying was to a target moisture content of 0.5% w/w (by Loss on drying); whereas when the Glatt GPGC30 fluid bed granulator was used, the drying was to a target moisture content of about 0.1-0.2% w/w (by Loss on drying). The granules were allowed to cool down and were then sieved through a 0.95mm sieve.

The resulting granulate, Carbomer 971 P, Carbomer 71 G, hydroxypropylmethylcellulose (HPMC 2208100000 mPa.s), microcrystalline cellulose , silicified microcrystalline cellulose , colloidal anhydrous silica, and magnesium stearate were weighed separately and sieved together through a 0.95mm sieve. The sieved materials were blended in a Turbula mixer for 5 min or in a bin blender for 10 min.

### Immediate Release Granulate Preparation:

The compound of formula (I-X) as a hemihydrate, microcrystalline cellulose (AVICEL PH 102), anhydrous lactose, and croscarmellose sodium (AC-Di-SOL) were screened through a sieve and loaded in a Glatt GPCG60 fluid bed granulator (Glatt). The powders were granulated with an aqueous solution of hydroxypropyl cellulose (KLUCEL EXF; binder concentration of 5% w/w solids) sprayed through a 1.8mm nozzle. The moisture level was monitored during the process, with samples taken every 10 minutes of the process. A moisture balance was used to determine loss on drying (LOD). The granules thus obtained were dried in the same equipment, to a target moisture content of 1.8% w/w (by loss on drying). The granules were allowed to cool down and were then screened together with the magnesium stearate. The resulting material was blended for 5min in a Bohle mixer.

### Tablet Formation:

Tablet manufacturing was performed on a single punch tablet press (Courtoy) equipped with oblong punches. The first layer compressed was the extended release (metformin HCl containing) layer using the granules prepared as described above, then granules for the immediate release layer (containing the compound of formula (I-X)) prepared as described above was added and the combined material compressed to form the tablet.

### Tablet Coating

Bi-layer tablets TAB-7-1 and TAB-7-2 were film coated with coating powder white (PVA based Opadry® II, Colorcon) to a coating weight of 3% w/w of core weight. The coating powder was suspended in purified water at the concentration of 20% w/w of solids in the suspension. The suspension was then sprayed on the tablets in a coating pan, at a pan bed temperature of 42°C and the resulting tablets dried.

### Dissolution Characteristics

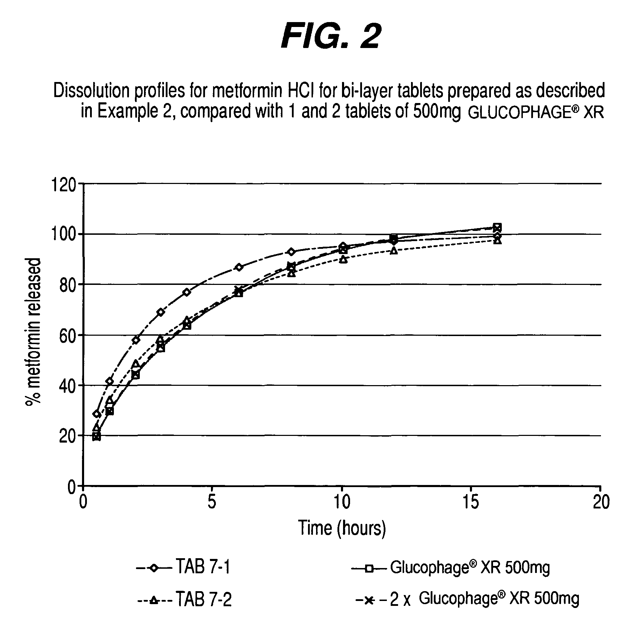

Figure 2 which follows herein illustrates the dissolution profiles measured (using Apparatus II, using the same conditions as described in Example 1, above) for the above prepared bi-layer tablets, comparing the dissolution profile of the metformin HCl portion of the bi-layer tablet with GLUCOPHAGE^{®} XR reference tablet(s). The results are displayed as an average for n=6 tablets tested.

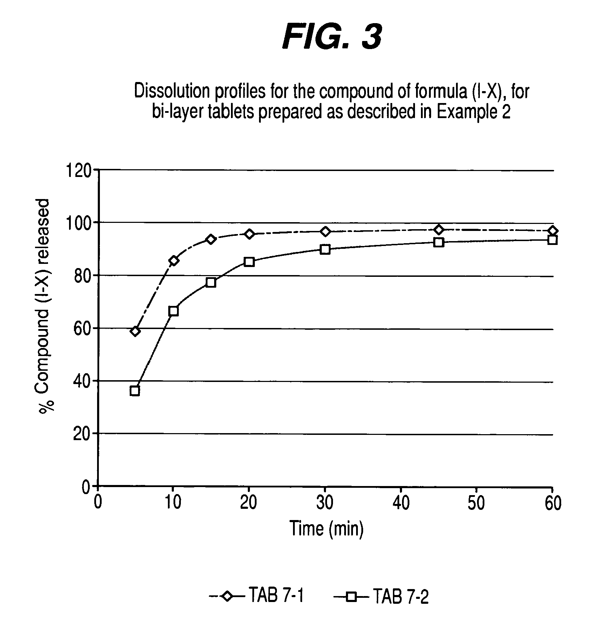

Dissolution profiles (using Apparatus I) were also measured for the above prepared bi-layer tablets to determine the dissolution of the compound of formula (I-X) portion of the bi-layer tablet, with results as illustrated in Figure 3 which follows herein. The dissolution of the compound of formula (I-X) was measured using USP apparatus I, 200 rpm rotation speed, in 900 mL of 0.5% POLYSORBATE 20 in water. The results are displayed as an average for n=6 tablets tested.

### Example 3

### Bi-layer Tablet Pharmaceutical Compositions

Two bi-layer tablet compositions comprising an immediate release portion containing the compound of formula (I-X) hemihydrate and an extended release portion containing metformin HCl, were prepared as described in Example 2, above, substituting components and amounts as indicated in the Tables below. The extended release (metformin HCl containing) portion of the tablet further comprised an internal phase granule and an extra-granular phase, which are combined to form the extended release compression mixture. The formulation / layer components and amounts in each said component within the tablet formulation and layers were as listed in Tables 3A and 3B, below.

**Table 3A: Metformin HCl Containing Composition Components**

| **Component** | **TAB-8-1** | **TAB-8-2** |
|---|---|---|
| **Extended Release Layer - Internal phase granule** | | |
| Metformin HCl | 500 mg | 500 mg |
| Hydroxypropylmethylcellulose (HPMC 2910 5mPa.s) | 7.5 mg | 7.5 mg |

| **Extended Release Layer - Extra-Granular Phase** | | |
|---|---|---|
| Carbomer 971P | 78 mg | 96 mg |
| Carbomer 71G | 26 mg | 32 mg |
| Hydroxypropylmethylcellulose (HPMC 2208 100,000 cps) | 195 mg | 240 mg |
| Silicified Microcrystalline Cellulose | 448 mg | 668.5 mg |
| Microcrystalline Cellulose | 32.5 mg | 40 mg |
| Colloidal Anhydrous Silica | 6.5 mg | 8 mg |
| Magnesium stearate | 6.5 mg | 8 mg |

**Table 3B: Compound of Formula (I-X) Containing Laver Components**

| **Component** | **Amounts** |
|---|---|
| Compound of Formula (I-X) as a hemihydrate | 153 mg |
| Microcrystalline cellulose (MCC: AVICEL PH 102) | 58.89 mg |
| Lactose Anhydrous Direct Tableting | 58.89 mg |
| Hydroxypropylcellulose (KLUCEL EXF) | 9 mg |
| Croscamellose Sodium (AC-Di-SOL) | 18 mg |
| Magnesium Stearate (Vegetable) | 2.22 mg |

### Dissolution Characteristics

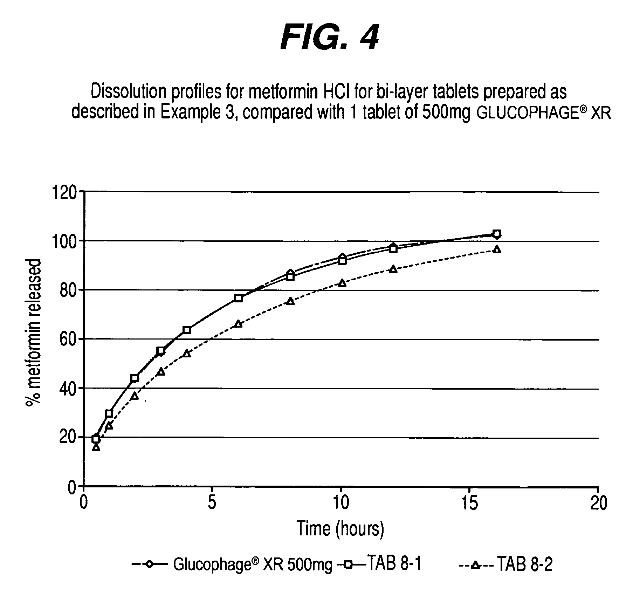

Dissolution profiles were measured (with Apparatus II according to the conditions displayed for Example 1) for the above prepared bi-layer tablets, comparing the dissolution of the metformin HCl portion of the bi-layer tablet with 1 tablet 500 mg GLUCOPHAGE^{®} XR, as illustrated in Figure 4, which follows herein. The data presented in Figure 4 and Table 3C indicate similar average dissolution profiles for the prepared tablets and the GLUCOPHAGE^{®} XR 500 mg comparator tablet.

**Table 3C: Measured Dissolution (in % Metformin Released)**

| **Time (hours)** | **500 mg Tablet GLUCOPHAGE^{®} XR** | **TAB-8-1** | **TAB-8-2** |
|---|---|---|---|
| 0.5 | 20 | 19 | 16 |
| 1 | 30 | 30 | 25 |
| 2 | 44 | 44 | 37 |
| 3 | 55 | 55 | 47 |
| 4 | 64 | 64 | 54 |
| 6 | 77 | 77 | 66 |
| 8 | 87 | 86 | 76 |
| 10 | 94 | 92 | 83 |
| 12 | 98 | 97 | 89 |

### Example 4

### Bi-Layer Tablet Pharmaceutical Composition

Two bi-layer tablet compositions were prepared comprising an immediate release layer comprising the compound of formula (I-X) and an extended release layer comprising metformin HCl. The composition of the immediate release and extended release layers are as listed in Tables 4A and 4B, below.

**Table 4A: Metformin HCl Containing Composition Components**

| **Component** | **TAB-9-1** | **TAB-9-2** | **%w/w** |
|---|---|---|---|
| **Extended Release Layer - Internal phase granule** | | | |
| Metformin HCl | 500 mg | 1000 mg | 76.9 |
| Lactose Monohydrate | 49.40 mg | 98.80 mg | 7.6 |
| CARBOPOL 971 P | 48.75 mg | 97.50 mg | 7.5 |

| **Extended Release Layer - Extra-Granule Phase** | | | |
|---|---|---|---|
| CARBOPOL71G | 48.75 mg | 97.50 mg | 7.5 |
| Magnesium Stearate | 3.25 mg | 6.50 mg | 0.5 |

**Table 4B: Compound of Formula (I-X) Containing Layer Components**

| **Component** | **TAB-9-1** | **TAB-9-2** | **% w/w** |
|---|---|---|---|
| Compound of Formula (I-X), hemihydrate | 51 mg | 153 mg | 51 |
| Lactose Anhydrate Direct Tableting | 19.63 mg | 58.89 mg | 19.6 |
| Microcrystalline cellulose | 19.63 mg | 58.89 mg | 19.6 |
| (AVICEL PH102) | | | |
| Hydroxypropylcellulose (KLUCEL EXF) | 3 mg | 9 mg | 3 |
| Croscarmellose Sodium (Ac-Di-Sol) | 6 mg | 18 mg | 6 |
| Magnesium Stearate | 0.74 mg | 2.22 mg | 0.74 |

The extended release layer comprising the metformin HCL was prepared as follows. Metformin HCl, lactose and CARBOPOL 971 P were placed in a fluid bed granulator, granulated and dried, to yield the internal phase granule. The resulting internal phase granule was then screened through #20 mesh. To the screened internal phase granule were then added CARBOPOL 71 G and magnesium stearate, and the resulting mixture blended to yield the extended release compression mixture. The compression mixture was then pressed to yield an extended release tablet layer.

The immediate release layer comprising the compound of formula (I-X) was prepared by mixing the compound of formula (I-X), the lactose, the microcrystalline cellulose and the croscarmellose sodium and then screening the resulting mixture. The screened mixture was then added to a fluid bed granulator, along with the hydropropyl celloluse (which was added as an aqueous solution). The resulting granules were dried in the granulator, then milled. The milled granules were then blended with pre-screened magnesium stearate by lubricant blending. The immediate release granules were then compressed with the previously prepared extended release tablet layer, to form a bi-layer tablet composition.

The bi-layer tablet composition was then coated with an aqueous suspension of OPADRY® II, to yield the final, coated, bi-layer tablet composition.

While the foregoing specification teaches the principles of the present invention, with examples provided for the purpose of illustration, it will be understood that the practice of the invention encompasses all of the usual variations, adaptations and/or modifications as come within the scope of the following claims and their equivalents.

## Claims

1. A pharmaceutical composition
wherein the pharmaceutical composition is a bi-layer tablet comprising
(a) an extended release layer comprising metformin hydrochloride and a controlled release excipient comprising a mixture of two carbomers; and
(b) an immediate release layer comprising a crystalline hemihydrate form of a compound of formula (I-X) 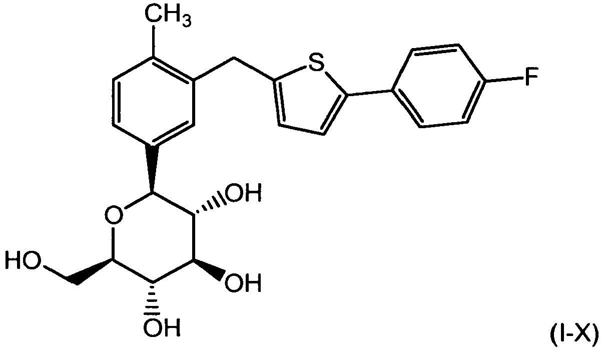 or pharmaceutically acceptable salt thereof.

2. A pharmaceutical composition as in Claim 1, wherein the metformin hydrochloride is present in an amount in the range of from about 250 mg to about 1500 mg.

3. A pharmaceutical composition as in Claim 1, wherein the immediate release layer further comprises one or more excipients selected from the group consisting of microcrystalline cellulose, lactose anhydrate, croscamellose sodium, hydroxypropylcellulose and magnesium stearate.

4. A pharmaceutical composition as in Claim 1, wherein the immediate release layer comprises
(a) the crystalline hemihydrate form of the compound of formula (I-X) as its corresponding crystalline hemihydrate in an amount of about 153 mg;
(b) microcrystalline cellulose, in amount of about 59 mg;
(c) lactose anhydrate in an amount of about 59 mg
(d) hydroxypropylcellulose in an amount of about 9 mg;
(e) croscamellose sodium in an amount of about 18 mg; and
(f) magnesium stearate in an amount of about 2.2 mg.

5. A pharmaceutical composition as in Claim 1, wherein the extended release layer further comprises
(a) an internal phase granule comprising the metformin HCl and one or more pharmaceutically acceptable excipients; and
(b) an extra-granular phase comprising one or more pharmaceutically acceptable excipients and no metformin hydrochloride.

6. A pharmaceutical composition as in Claim 1, wherein the two carbomers are CARBOMER 971 P in an amount of about 78 mg; CARBOMER 71 G in an amount of about 26 mg.

7. A pharmaceutical composition as in Claim 1, wherein the extended release layer comprises
(a) an internal phase granule comprising metformin HCl in an amount of about 500 mg and hydroxypropylmethylcellulose in an amount of about 7.5 mg; and
(b) an extra-granular phase comprising CARBOMER 971 P in an amount of about 78 mg; CARBOMER 71 G in an amount of about 26 mg; and hydroxypropylmethylcellulose in an amount of about 195 mg.

8. A pharmaceutical composition as in Claim 7, wherein the extra-granular phase of the extended release layer comprises further comprises silicified microcrystalline cellulose in an amount of about 448 mg; microcrystalline cellulose in an amount of about 32.5 mg; colloidal anhydrous silica in an amount of about 6.5 mg; and magnesium stearate in an amount of about 6.5 mg.

9. A pharmaceutical composition as in Claim 1, wherein about 75% of the metformin hydrochloride is released within about 5 hours; and wherein greater than about 90% of the metformin HCl is released within about 12 hours as measured using 1,000 mL 0.05M phosphate buffer pH 6.8, Apparatus II (Paddle) at 100 rpm (USP test n°8).

10. A pharmaceutical composition as in Claim 1, wherein at least about 85% of the metformin hydrochloride is released within about 10 hours as measured using 1,000 mL 0.05M phosphate buffer pH 6.8, Apparatus II (Paddle) at 100 rpm (USP test n°8).

11. A pharmaceutical composition as in Claim 1, wherein between about 25% and about 45% of the metformin hydrochloride is released within about 1 hour; wherein between about 50% and about 70% of the metformin hydrochloride is released within about 3 hours; and wherein at least about 80% of the metformin hydrochloride is released within about 10 hours as measured using 1,000 mL 0.05M phosphate buffer pH 6.8, Apparatus II (Paddle) at 100 rpm (USP test n°8).

12. A pharmaceutical composition as in Claim 1, wherein
the extended release layer comprises an internal phase granule comprising about 500 mg of metformin HCl and about 7.5 mg of, hydroxypropylmethylcellulose 5mPa.s; and an extra-granular phase comprising the mixture of two carbomers and about 195 mg of hydroxypropylmethylcellulose 100,000 mPa.s; and
wherein the immediate release layer comprises the crystalline hemihydrate form of the compound of formula (I-X), in an amount of about 153 mg; and
wherein at least about 80% of the metformin HCl is released within about 10 hours of administration as measured using 1,000 mL 0.05M phosphate buffer pH 6.8, Apparatus II (Paddle) at 100 rpm (USP test n°8).

13. A composition of Claim 1 for use in a method of treating a glucose related disorder.

14. A composition of Claim 1 for use in a method as in Claim 13, wherein the glucose related disorder is selected from the group consisting of diabetes mellitus, diabetic retinopathy, diabetic neuropathy, diabetic nephropathy, delayed wound healing, insulin resistance, hyperglycemia, hyperinsulinemia, elevated blood levels of fatty acids, elevated blood levels of glucose, hyperlipidemia, obesity, hypertriglyceridemia, Syndrome X, diabetic complications, atherosclerosis and hypertension.

15. A composition of Claim 1 for use in a method as in Claim 13, wherein the glucose related disorder is type 2 diabetes mellitus.

## Patentansprüche

1. Pharmazeutische Zusammensetzung,
wobei es sich bei der pharmazeutischen Zusammensetzung um eine zweischichtige Tablette handelt, umfassend
(a) eine Retard-Schicht, umfassend Metforminhydrochlorid und einen Exzipienten für kontrollierte Freisetzung, umfassend eine Mischung von zwei Carbomeren, und
(b) eine Schicht mit sofortiger Freisetzung, umfassend eine kristalline Hemihydratform einer Verbindung der Formel (I-X) 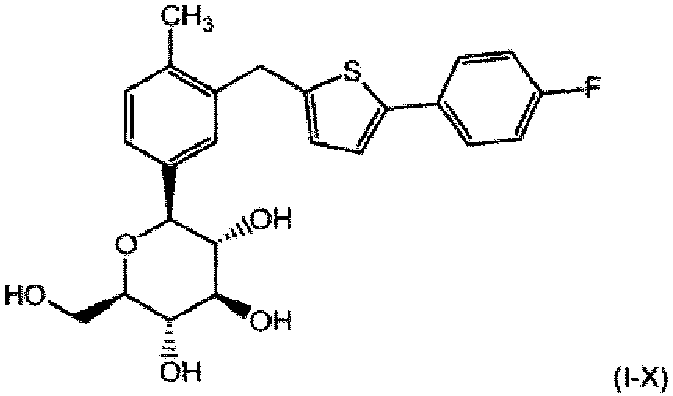 oder ein pharmazeutisch unbedenkliches Salz davon.

2. Pharmazeutische Zusammensetzung nach Anspruch 1, wobei das Metforminhydrochlorid in einer Menge im Bereich von etwa 250 mg bis etwa 1500 mg vorliegt.

3. Pharmazeutische Zusammensetzung nach Anspruch 1, wobei die Schicht mit sofortiger Freisetzung weiterhin einen oder mehrere Exzipienten ausgewählt aus der Gruppe bestehend aus mikrokristalliner Cellulose, Lactoseanhydrat, Croscarmellose-Natrium, Hydroxypropylcellulose und Magnesiumstearat umfasst.

4. Pharmazeutische Zusammensetzung nach Anspruch 1, wobei die Schicht mit sofortiger Freisetzung Folgendes umfasst:
(a) die kristalline Hemihydratform der Verbindung der Formel (I-X) als ihr entsprechendes kristallines Hemihydrat in einer Menge von etwa 153 mg,
(b) mikrokristalline Cellulose in einer Menge von etwa 59 mg,
(c) Lactoseanhydrat in einer Menge von etwa 59 mg,
(d) Hydroxypropylcellulose in einer Menge von etwa 9 mg,
(e) Croscamellose-Natrium in einer Menge von etwa 18 mg und
(f) Magnesiumstearat in einer Menge von etwa 2,2 mg.

5. Pharmazeutische Zusammensetzung nach Anspruch 1, wobei die Retard-Schicht weiterhin Folgendes umfasst:
(a) ein Innere-Phase-Granulat, welches das Metformin-HCl und einen oder mehrere pharmazeutisch unbedenkliche Exzipienten umfasst, und
(b) eine extragranuläre Phase, welche einen oder mehrere pharmazeutisch unbedenkliche Exzipienten und kein Metforminhydrochlorid umfasst.

6. Pharmazeutische Zusammensetzung nach Anspruch 1, wobei es sich bei den beiden Carbomeren um CARBOMER 971P in einer Menge von etwa 78 mg und CARBOMER 71G in einer Menge von etwa 26 mg handelt.

7. Pharmazeutische Zusammensetzung nach Anspruch 1, wobei die Retard-Schicht Folgendes umfasst:
(a) ein Innere-Phase-Granulat, das Metformin-HCl in einer Menge von etwa 500 mg und Hydroxypropylmethylcellulose in einer Menge von etwa 7,5 mg umfasst, und
(b) eine extragranuläre Phase, die CARBOMER 971P in einer Menge von etwa 78 mg, CARBOMER 71G in einer Menge von etwa 26 mg und Hyroxypropylmethylcellulose in einer Menge von etwa 195 mg umfasst.

8. Pharmazeutische Zusammensetzung nach Anspruch 7, wobei die extragranuläre Phase der Retard-Schicht weiterhin silizifierte mikrokristalline Cellulose in einer Menge von etwa 448 mg, mikrokristalline Cellulose in einer Menge von etwa 32,5 mg, kolloidales wasserfreies Siliciumdioxid in einer Menge von etwa 6,5 mg und Magnesiumstearat in einer Menge von etwa 6,5 mg umfasst.

9. Pharmazeutische Zusammensetzung nach Anspruch 1, wobei etwa 75% des Metforminhydrochlorids innerhalb von etwa 5 Stunden freigesetzt werden und wobei mehr als etwa 90% des Metformin-HCl innerhalb von etwa 12 Stunden freigesetzt werden, gemessen unter Verwendung von 1000 ml 0,05 M Phosphatpuffer pH-Wert 6,8, Apparat II (Paddle) bei 100 U/min (USP-Test Nr. 8).

10. Pharmazeutische Zusammensetzung nach Anspruch 1, wobei mindestens etwa 85% des Metforminhydrochlorids innerhalb von etwa 10 Stunden freigesetzt werden, gemessen unter Verwendung von 1000 ml 0,05 M Phosphatpuffer pH-Wert 6,8, Apparat II (Paddle) bei 100 U/min (USP-Test Nr. 8).

11. Pharmazeutische Zusammensetzung nach Anspruch 1, wobei zwischen etwa 25% und etwa 45% des Metforminhydrochlorids innerhalb etwa 1 Stunde freigesetzt werden, wobei zwischen etwa 50% und etwa 70% des Metforminhydrochlorids innerhalb von etwa 3 Stunden freigesetzt werden und wobei mindestens etwa 80% des Metforminhydrochlorids innerhalb von etwa 10 Stunden freigesetzt werden, gemessen unter Verwendung von 1000 ml 0,05 M Phosphatpuffer pH-Wert 6,8, Apparat II (Paddle) bei 100 U/min (USP-Test Nr. 8).

12. Pharmazeutische Zusammensetzung nach Anspruch 1, wobei
die Retard-Schicht ein Innere-Phase-Granulat, welches etwa 500 mg Metformin-HCl und etwa 7,5 mg Hydroxypropylmethylcellulose 5 mPa.s umfasst, und eine extragranuläre Phase, welche die Mischung von zwei Carbomeren und etwa 195 mg Hydroxypropylmethylcellulose 100 000 mPa.s umfasst, umfasst und
wobei die Schicht mit sofortiger Freisetzung die kristalline Hemihydratform der Verbindung der Formel (I-X) in einer Menge von etwa 153 mg umfasst und
wobei mindestens etwa 80% des Metformin-HCl innerhalb von etwa 10 Stunden nach Verabreichung freigesetzt werden, gemessen unter Verwendung von 1000 ml 0,05 M Phosphatpuffer pH-Wert 6,8, Apparat II (Paddle) bei 100 U/min (USP-Test Nr. 8).

13. Zusammensetzung nach Anspruch 1 zur Verwendung in einem Verfahren zur Behandlung einer mit Glucose in Zusammenhang stehenden Erkrankung.

14. Zusammensetzung nach Anspruch 1 zur Verwendung in einem Verfahren nach Anspruch 13, wobei die mit Glucose in Zusammenhang stehende Erkrankung ausgewählt ist aus der Gruppe bestehend aus Diabetes mellitus, diabetischer Retinopathie, diabetischer Neuropathie, diabetischer Nephropathie, einer verzögerten Wundheilung, Insulinresistenz, Hyperglykämie, Hyperinsulinämie, erhöhten Fettsäure-Blutspiegeln, erhöhten Glucose-Blutspiegeln, Hyperlipidämie, Obesitas, Hypertriglyzeridämie, Syndrom X, diabetischen Komplikationen, Atherosklerose und Hypertonie.

15. Zusammensetzung nach Anspruch 1 zur Verwendung in einem Verfahren nach Anspruch 13, wobei es sich bei der mit Glucose in Zusammenhang stehenden Erkrankung um Typ-2-Diabetes mellitus handelt.

## Revendications

1. Composition pharmaceutique en comprimé bicouche comprenant :
a) une couche à libération prolongée contenant du chlorhydrate de metformine et un excipient à libération contrôlée comprenant un mélange de deux carbomères ; et
b) une couche à libération immédiate contenant une forme hémihydrate cristalline d'un composé de formule (I-X) 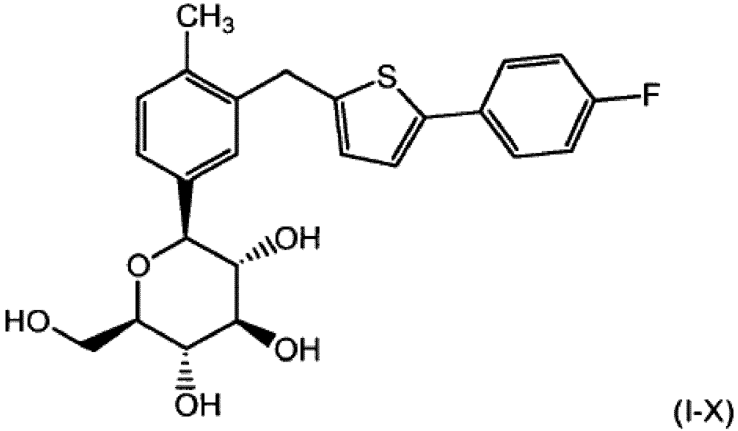 ou un de ses sels pharmaceutiquement acceptables.

2. Composition pharmaceutique selon la revendication 1, dans laquelle le chlorhydrate de metformine est présent à hauteur d'entre environ 250 mg et environ 1 500 mg.

3. Composition pharmaceutique selon la revendication 1, dans laquelle la couche à libération immédiate contient en outre un ou plusieurs excipients choisis dans le groupe constitué de la cellulose microcristalline, le lactose anhydre, le croscarmellose sodique, l'hydroxypropylcellulose et le stéarate de magnésium.

4. Composition pharmaceutique selon la revendication 1, dans laquelle la couche à libération immédiate contient :
a) la forme hémihydrate cristalline du composé de formule (I-X) sous sa forme hémihydrate cristalline à hauteur d'environ 153 mg ;
b) la cellulose microcristalline à hauteur d'environ 59 mg ;
c) le lactose anhydre à hauteur d'environ 59 mg ;
d) l'hydroxypropylcellulose à hauteur d'environ 9 mg ;
e) le croscarmellose sodique à hauteur d'environ 18 mg ;
f) le stéarate de magnésium à hauteur d'environ 2,2 mg.

5. Composition pharmaceutique selon la revendication 1, dans laquelle la couche à libération prolongée comprend en outre :
a) une phase intragranulaire contenant le chlorhydrate de metformine et un ou plusieurs excipients pharmaceutiquement acceptables ; et
b) une phase extragranulaire contenant un ou plusieurs excipients pharmaceutiquement acceptables sans chlorhydrate de metformine.

6. Composition pharmaceutique selon la revendication 1, dans laquelle les deux carbomères sont CARBOMER 971P à hauteur d'environ 78 mg ; CARBOMER 71G à hauteur d'environ 26 mg.

7. Composition pharmaceutique selon la revendication 1, dans laquelle la couche à libération prolongée contient :
a) une phase intragranulaire contenant le chlorhydrate de metformine à hauteur d'environ 500 mg et de l'hydroxypropylméthylcellulose à hauteur d'environ 7,5 mg ; et
b) une phase extragranulaire contenant CARBOMER 971P à hauteur d'environ 78 mg ; CARBOMER 71G à hauteur d'environ 26 mg ; et de l'hydroxypropylcellulose à hauteur d'environ 195 mg.

8. Composition pharmaceutique selon la revendication 7, dans laquelle la phase extragranulaire de la couche à libération prolongée contient en outre de la cellulose microcristalline silicifiée à hauteur d'environ 448 mg, de la cellulose microcristalline à hauteur d'environ 32,5 mg, de la silice anhydre colloïdale à hauteur d'environ 6,5 mg et du stéarate de magnésium à hauteur d'environ 6,5 mg.

9. Composition pharmaceutique selon la revendication 1, où environ 75 % du chlorhydrate de metformine est libéré sous environ 5 heures, et où plus de 90 % du chlorhydrate de metformine est libéré sous environ 12 heures tel que mesuré en utilisant 1 000 ml de tampon phosphate 0,05 M pH 6,8, un USP Apparatus II (Palette) à 100 tr/min (Test USP n°8).

10. Composition pharmaceutique selon la revendication 1, où au moins environ 85 % du chlorhydrate de metformine est libéré sous environ 10 heures tel que mesuré en utilisant 1 000 ml de tampon phosphate 0,05 M pH 6,8, un USP Apparatus II (Palette) à 100 tr/min (Test USP n°8).

11. Composition pharmaceutique selon la revendication 1, où entre environ 25 % et environ 45 % du chlorhydrate de metformine est libéré sous environ 1 heure ; où entre environ 50 % et environ 70 % du chlorhydrate de metformine est libéré sous environ 3 heures ; et où environ 80 % du chlorhydrate de metformine est libéré sous environ 10 heures tel que mesuré en utilisant 1 000 ml de tampon phosphate 0,05 M pH 6,8, un USP Apparatus II (Palette) à 100 tr/min (Test USP n°8).

12. Composition pharmaceutique selon la revendication 1, où la couche à libération immédiate contient une phase intragranulaire comprenant environ 500 mg de chlorhydrate de metformine et environ 7,5 mg d'hydroxypropylméthylcellulose 5 mPa.s, et une phase extragranulaire comprenant le mélange de deux carbomères et environ 195 mg d'hydroxypropylméthylcellulose 100 000 mPa.s ; et
dans laquelle la couche à libération immédiate contient la forme hémihydrate cristalline du composé de formule (I-X) à hauteur d'environ 153 mg ; et
dans laquelle au moins environ 80 % du chlorhydrate de metformine est libéré sous environ 10 heures après administration, tel que mesuré en utilisant 1 000 ml de tampon phosphate 0,05 M pH 6,8, un USP Apparatus II (Palette) à 100 tr/min (Test USP n°8).

13. Composition selon la revendication 1 pour utilisation dans un procédé de traitement d'un trouble lié au glucose.

14. Composition selon la revendication 1 pour utilisation dans un procédé selon la revendication 13, dans laquelle le trouble lié au glucose est choisi dans le groupe constitué du diabète sucré, de la rétinopathie diabétique, de la neuropathie diabétique, de la néphropathie diabétique, du retard à la cicatrisation, de la résistance à l'insuline, de l'hyperglycémie, de l'hyperinsulinémie, des niveaux sanguins élevés en acides gras, des niveaux élevés de glycémie, de l'hyperlipidémie, de l'obésité, de l'hypertriglycéridémie, du syndrome X, des complications du diabète, de l'athérosclérose et de l'hypertension.

15. Composition selon la revendication 1 pour utilisation dans un procédé selon la revendication 13, dans laquelle le trouble lié au glucose est le diabète sucré de type 2.
